# EUROPEAN PATENT APPLICATION

(11) **EP 1 975 828 A2**
(43) Date of publication of application: **01.10.2008**
(21) Application number: 08251178.3
(22) Date of filing: 28.03.2008
(51) Int. Cl.: G06F 19/00

(54) **Configuring software for effective health monitoring or the like**

(30) Priority: 30.03.2007 US 731778; 30.03.2007 US 731801; 30.03.2007 US 731745
(71) Applicant: Searete LLC., Bellevue, WA 98004 (US)
(72) Inventor: Jung, Edward K. Y., Bellevue, WA 98005-1403 (US); Leuthardt, Eric C., St Louis, MO 63130 (US); Levien, Royce A., Lexington, MA 02420 (US); Lord, Robert W., Seattle, WA 98112 (US); Malamud, Mark A., Seattle, WA 98119 (US)
(74) Representative: Harris, Ian Richard

(57) **Abstract**

A system, method, computer program product, and carrier are described for obtaining an indication of an activity status change of an application program and causing a movement of data distillation code relating to physiology-indicative data in response to the indication of the activity status change of the application program; or for obtaining (a) first clinical data acceptable to a clinical analysis module and (b) a pointer to the clinical analysis module, configuring one or more applications using the pointer to the clinical analysis module after receiving at least the first clinical data acceptable to the clinical analysis module, and using second clinical data acceptable to the clinical analysis module with at least one of the one or more applications configured using the pointer to the clinical analysis module after receiving at least the first clinical data acceptable to the clinical analysis module.

## Description

### CROSS-REFERENCE TO RELATED APPLICATIONS

### Related Applications:

United States Patent Application No. 11/731,778, entitled EFFECTIVE LOW-PROFILE HEALTH MONITORING OR THE LIKE, naming Edward K.Y. Jung, Eric C. Leuthardt; Royce A. Levien, Robert W. Lord and Mark A. Malamud as inventors, filed 30 March 2007.

United States Patent Application No. 11/731,801, entitled effective low-profile health MONITORING OR THE LIKE, naming Edward K.Y. Jung, Eric C. Leuthardt; Royce A. Levien, Robert W. Lord and Mark A. Malamud as inventors, filed 30 March 2007.

United States Patent Application No. 11/731,745, entitled EFFECTIVE RESPONSE PROTOCOLS FOR HEALTH MONITORING OR THE LIKE, naming Edward K.Y. Jung, Eric C. Leuthardt; Royce A. Levien, Robert W. Lord and Mark A. Malamud as inventors, filed 30 March 2007.

All subject matter of the Related Applications and of any and all parent, grandparent, great-grandparent, etc. applications of the Related Applications is incorporated herein by reference to the extent such subject matter is not inconsistent herewith.

### SUMMARY

In one aspect, a method includes but is not limited to obtaining data from occupational or leisure intercommunication at least between a device and a user and signaling a data distillation indicating a health status change of the user at least partly based on the data from the occupational or leisure intercommunication. In addition to the foregoing, other method aspects are described in the claims, drawings, and text forming a part of the present disclosure.

In one or more various aspects, related systems include but are not limited to circuitry and/or programming for effecting the herein-referenced method aspects; the circuitry and/or programming can be virtually any combination of hardware, software, and/or firmware configured to effect the herein-referenced method aspects depending upon the design choices of the system designer.

In one aspect, a system includes but is not limited to circuitry for obtaining data from occupational or leisure intercommunication at least between a device and a user and circuitry for signaling a data distillation indicating.a health status change of the user at least partly based on the data from the occupational or leisure intercommunication. In addition to the foregoing, other system aspects are described in the claims, drawings, and text forming a part of the present disclosure.

In one aspect, a method includes but is not limited to receiving health-status-indicative data surreptitiously captured from an interaction between a device and a user and applying one or more data extraction criteria to the health-status-indicative data surreptitiously captured from the interaction between the device and the user. In addition to the foregoing, other method aspects are described in the claims, drawings, and text forming a part of the present disclosure.

In one or more various aspects, related systems include but are not limited to circuitry and/or programming for effecting the herein referenced method aspects; the circuitry and/or programming can be virtually any combination of hardware, software, and/or firmware configured to effect the herein referenced method aspects depending upon the design choices of the system designer.

In one aspect, a system includes but is not limited to circuitry for receiving health-status-indicative data surreptitiously captured from an interaction between a device and a user and circuitry for applying one or more data extraction criteria to the health-status-indicative data surreptitiously captured from the interaction between the device and the user. In addition to the foregoing, other system aspects are described in the claims, drawings, and text forming a part of the present disclosure.

In one aspect, a method includes but is not limited to obtaining an indication of an activity status change of an application program and causing a movement of data distillation code relating to physiology-indicative data in response to the indication of the activity status change of the application program. In addition to the foregoing, other method aspects are described in the claims, drawings, and text forming a part of the present disclosure.

In one or more various aspects, related systems include but are not limited to circuitry and/or programming for effecting the herein referenced method aspects; the circuitry and/or programming can be virtually any combination of hardware, software, and/or firmware configured to effect the herein referenced method aspects depending upon the design choices of the system designer.

In one aspect, a system includes but is not limited to circuitry for obtaining an indication of an activity status change of an application program and circuitry for causing a movement of data distillation code relating to physiology-indicative data in response to the indication of the activity status change of the application program. In addition to the foregoing, other system aspects are described in the claims, drawings, and text forming a part of the present disclosure.

In one aspect, a method includes but is not limited to obtaining (a) first clinical data acceptable to a clinical analysis module and (b) a pointer to the clinical analysis module, configuring one or more applications using the pointer to the clinical analysis module after receiving at least the first clinical data acceptable to the clinical analysis module, and using second clinical data acceptable to the clinical analysis module with at least one of the one or more applications configured using the pointer to the clinical analysis module after receiving at least the first clinical data acceptable to the clinical analysis module. In addition to the foregoing, other method aspects are described in the claims, drawings, and text forming a part of the present disclosure.

In one or more various aspects, related systems include but are not limited to circuitry and/or programming for effecting the herein referenced method aspects; the circuitry and/or programming can be virtually any combination of hardware, software, and/or firmware configured to effect the herein referenced method aspects depending upon the design choices of the system designer.

In one aspect, a system includes but is not limited to circuitry for obtaining (a) first clinical data acceptable to a clinical analysis module and (b) a pointer to the clinical analysis module, circuitry for configuring one or more applications using the pointer to the clinical analysis module after receiving at least the first clinical data acceptable to the clinical analysis module, and circuitry for using second clinical data acceptable to the clinical analysis module with at least one of the one or more applications configured using the pointer to the clinical analysis module after receiving at least the first clinical data acceptable to the clinical analysis module. In addition to the foregoing, other system aspects are described in the claims, drawings, and text forming a part of the present disclosure.

In one aspect, a method includes but is not limited to receiving an indication of an anomalous device-interactive performance of a specific individual and selecting one or more diagnostic instructions at least partly based on the anomalous device-interactive performance of the specific individual. In addition to the foregoing, other method aspects are described in the claims, drawings, and text forming a part of the present disclosure.

In one or more various aspects, related systems include but are not limited to circuitry and/or programming for effecting the herein referenced method aspects; the circuitry and/or programming can be virtually any combination of hardware, software, and/or firmware configured to effect the herein referenced method aspects depending upon the design choices of the system designer.

In one aspect, a system includes but is not limited to circuitry for receiving an indication of an anomalous device-interactive performance of a specific individual and circuitry for selecting one or more diagnostic instructions at least partly based on the anomalous device-interactive performance of the specific individual. In addition to the foregoing, other system aspects are described in the claims, drawings, and text forming a part of the present disclosure.

In one aspect, a method includes but is not limited to signaling a decision whether to notify a first party at least by applying a first screen to one or more health-status-indicative updates relating to a second party and signaling a decision whether to notify a third party at least by applying a second screen to the one or more health-status-indicative updates relating to the second party. In addition to the foregoing, other method aspects are described in the claims, drawings, and text forming a part of the present disclosure.

In one or more various aspects, related systems include but are not limited to circuitry and/or programming for effecting the herein referenced method aspects; the circuitry and/or programming can be virtually any combination of hardware, software, and/or firmware configured to effect the herein referenced method aspects depending upon the design choices of the system designer.

In one aspect, a system includes but is not limited to circuitry for signaling a decision whether to notify a first party at least by applying a first screen to one or more health-status-indicative updates relating to a second party and circuitry for signaling a decision whether to notify a third party at least by applying a second screen to the one or more health-status-indicative updates relating to the second party. In addition to the foregoing, other system aspects are described in the claims, drawings, and text forming a part of the present disclosure.

In addition to the foregoing, various other method and/or system and/or program product and/or physical carrier aspects are set forth and described in the teachings such as text (e.g., claims and/or detailed description) and/or drawings of the present disclosure.

The foregoing is a summary and thus contains, by necessity, simplifications, generalizations and omissions of detail; consequently, those skilled in the art will appreciate that the summary is illustrative only and is NOT intended to be in any way limiting. Other aspects, features, and advantages of the devices and/or processes and/or other subject matter described herein will become apparent in the teachings set forth herein.

### BRIEF DESCRIPTION OF THE FIGURES

Embodiments are described hereinafter, by way of example only, with reference to the drawings.
**FIG. 1** depicts an example environment in which one or more technologies may be implemented.
**FIG. 2** depicts a high-level logic flow of an operational process.
**FIG. 3** depicts an example environment in which one or more technologies may be implemented.
**FIG. 4** depicts a high-level logic flow of an operational process.
**FIG. 5** depicts an example environment in which one or more technologies may be implemented.
**FIG. 6** depicts a high-level logic flow of an operational process.
**FIG. 7** depicts an example environment in which one or more technologies may be implemented.
**FIG. 8** depicts a high-level logic flow of an operational process.
**FIG. 9** depicts an example environment in which one or more technologies may be implemented.
**FIG. 10** depicts a high-level logic flow of an operational process.
**FIG. 11** depicts an example environment in which one or more technologies may be implemented.
**FIG. 12** depicts a high-level logic flow of an operational process.
**FIGS. 13-20** depict example environments in which one or more technologies may be implemented.
**FIGS. 21-23** depict variants of the flow of **FIG. 2****.**
**FIGS. 24-25** depict variants of the flow of **FIG. 6****.**
**FIG. 26** depicts variants of the flow of **FIG. 8****.**
**FIGS. 27-28** depict variants of the flow of **FIG. 10****.**
**FIG. 29** depicts variants of the flow of **FIG. 12****.**
**FIG. 30** depicts variants of the flow of **FIG. 4****.**

### DETAILED DESCRIPTION

Those having skill in the art will recognize that the state of the art has progressed to the point where there is little distinction left between hardware and software implementations of aspects of systems; the use of hardware or software is generally (but not always, in that in certain contexts the choice between hardware and software can become significant) a design choice representing cost vs. efficiency tradeoffs. Those having skill in the art will appreciate that there are various vehicles by which processes and/or systems and/or other technologies described herein can be effected (e.g., hardware, software, and/or firmware), and that the preferred vehicle will vary with the context in which the processes and/or systems and/or other technologies are deployed. For example, if an implementer determines that speed and accuracy are paramount, the implementer may opt for a mainly hardware and/or firmware vehicle; alternatively, if flexibility is paramount, the implementer may opt for a mainly software implementation; or, yet again alternatively, the implementer may opt for some combination of hardware, software, and/or firmware. Hence, there are several possible vehicles by which the processes and/or devices and/or other technologies described herein may be effected, none of which is inherently superior to the other in that any vehicle to be utilized is a choice dependent upon the context in which the vehicle will be deployed and the specific concerns (e.g., speed, flexibility, or predictability) of the implementer, any of which may vary. Those skilled in the art will recognize that optical aspects of implementations will typically employ optically-oriented hardware, software, and or firmware.

In the following detailed description, reference is made to the accompanying drawings, which form a part hereof. The use of the same symbols in different drawings typically indicates similar or identical items. The illustrative embodiments described in the detailed description, drawings, and claims are not meant to be limiting. Other embodiments may be utilized, and other changes may be made, without departing from the spirit or scope of the subject matter presented here.

Following are a series of systems and flowcharts depicting implementations of processes. For ease of understanding, the flowcharts are organized such that the initial flowcharts present implementations via an initial "big picture" viewpoint and thereafter the following flowcharts present alternate implementations and/or expansions of the "big picture" flowcharts as either sub-steps or additional steps building on one or more earlier-presented flowcharts. Those having skill in the art will appreciate that the style of presentation utilized herein (e.g., beginning with a presentation of a flowchart(s) presenting an overall view and thereafter providing additions to and/or further details in subsequent flowcharts) generally allows for a rapid and easy understanding of the various process implementations. In addition, those skilled in the art will further appreciate that the style of presentation used herein also lends itself well to modular and/or object-oriented program design paradigms.

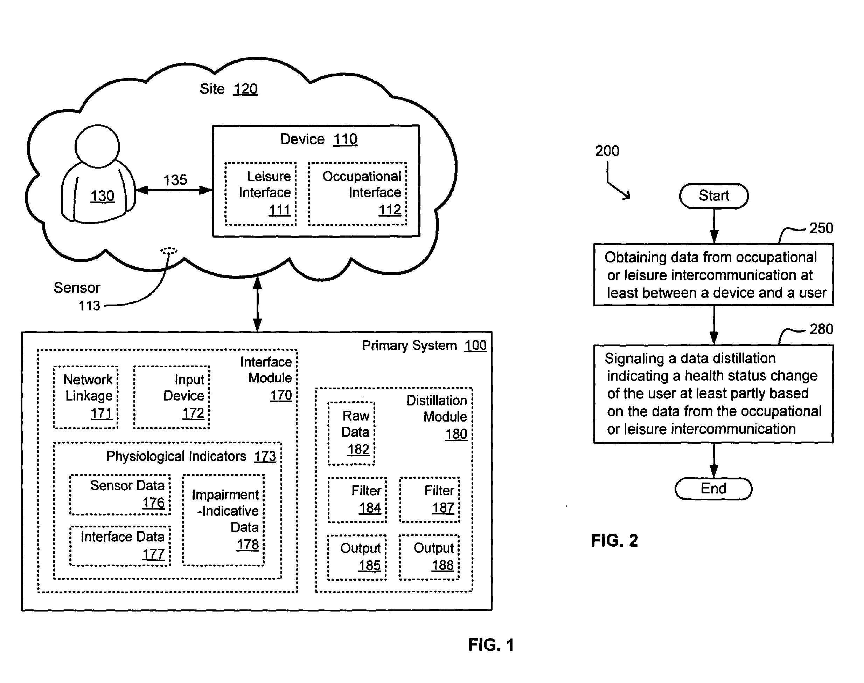

With reference now to **FIG. 1****,** shown is an example of a system that may serve as a context for introducing one or more processes and/or devices described herein. As shown primary system **100** can communicate with site **120** at which user **130** can have (or have had) intercommunication **135** with device **110.** Device **110** can include one or more of leisure interface **111** or occupational interface **112** to facilitate at least some occupational or leisure intercommunication detectable to one or more instances of sensor **113.**

As shown, primary system **100** can include one or more of interface module **170** or distillation module **180.** Interface module **170** can include one or more instances of network linkage **171,** input device **172,** or physiological indicators **173.** Such indicators can, for example, include one or more instances of sensor data **176** (from sensor **113,** e.g.), interface data **177,** or impairment-indicative data **178.** Distillation module **180** can include one or more instances of raw data **182,** filters **184, 187,** or outputs **185, 188.**

With reference now to **FIG. 2****,** there is shown a high-level logic flow **200** of an operational process. Flow **200** includes operation **250** obtaining data from occupational or leisure intercommunication at least between a device and a user (e.g. interface module **170** receiving sensor data **176** from sensor **113** or interface data **177** via device **110** at least from intercommunication 135). In some embodiments, sensor data **176** or interface data **177** thus obtained can also indicate other kinds of communications (with other parties or devices, e.g.) or other events. Alternatively or additionally, some or all of the data can be obtained in the form of raw data **182** as exemplified herein.

Flow **200** further includes operation **280** signaling a data distillation indicating a health status change of the user at least partly based on the data from the occupational or leisure intercommunication (e.g. distillation module **180** indicating substantial changes in impairment-indicative data **178** or other physiological indicators **173** relating to user **130**). In some embodiments, one or more instances of sensors **113** obtain data from site **120** while stationary, observing user 130 (and perhaps other visitors to site **120**). In some variants, one or more filters **184, 187** respectively provide one or more distillation outputs **185, 188** that may reasonably be expected to evaluate, consolidate, organize, or otherwise facilitate an interpretation of at least some of raw data **182** or other data. In a variant in which device **110** or sensor **113** includes a chemical or other biometric sensor operable to detect alcohol-containing breath, pale or clammy skin, a fast heartbeat or other difference relative to an earlier or later measurement, such changes can indicate a health status change susceptible of recordation and even automatic detection as described herein. Such signaling can be directed to one or more of user **130**, caregivers or third parties, an archiving system or other communication system, or the like.

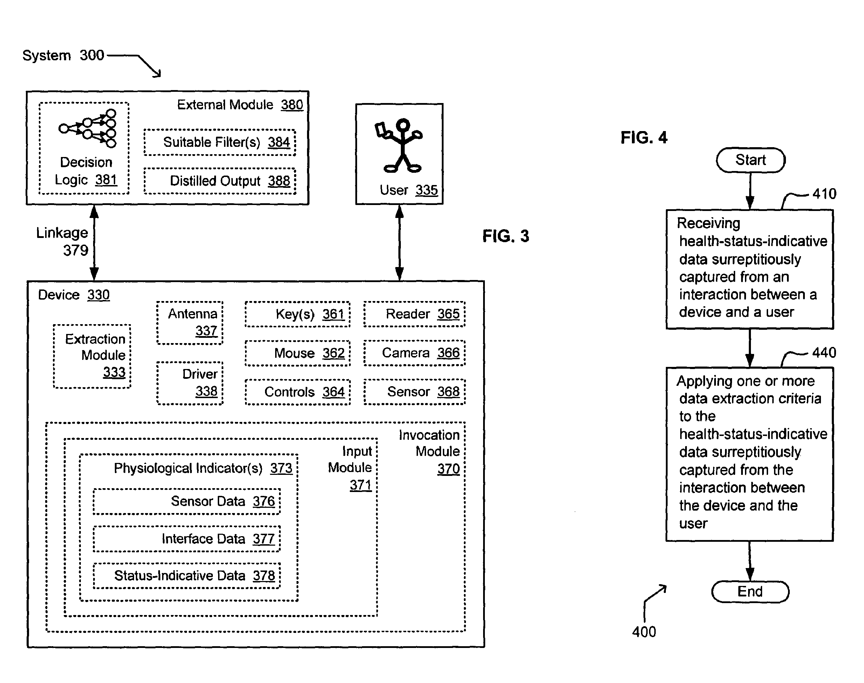

With reference now to **FIG. 3****,** shown is an example of a system that may serve as a context for introducing one or more processes and/or devices described herein. As shown system **300** includes device **330** able to interact with user **335** or with external module **380.** External module **380** can include one or more instances of decision logic **381,** suitable filters **384,** or distilled output **388.** In communicating with external module **380,** device **330** can optionally be configured to receive data through linkage **379** (via antenna **337,** e.g.) or send data through linkage **379** (via driver **338,** e.g.). Linkage **379** can include one or more conduits, wireless signal paths, or the like. Device **330** can likewise (optionally) include one or more instances of extraction module **333,** keys **361,** mouse **362,** controls **364,** readers **365,** cameras **366,** sensors **368,** or invocation modules **370.** Invocation module **370** can include input module(s) **371** with one or more physiological indicators **373** in the form of sensor data **376,** interface data **377,** status-indicative data **378,** or the like.

With reference now to **FIG. 4****,** there is shown a high-level logic flow **400** of an operational process. Flow **400** includes operation **410** receiving health-status-indicative data surreptitiously captured from an interaction between a device and a user (e.g. input module **371** or the like gathering data from a device interaction with user **335** engaged in everyday tasks or otherwise not consciously aware of the data being gathered). The health-status-indicative data can contain one or more physiological indicators **373,** for example, comprising sensor data **376,** interface data **377,** or other status-indicative data **378** that may relate to user **335.**

In some embodiments, such data is captured "surreptitiously" by virtue of the device participating in the interaction without a conspicuous notice of a capture event. (The user can authorize such surreptitious data captures before or after the capture events, for example.) Such modes of data acquisition can avoid error arising from consciousness of observation, for example, especially for situations in which a full-blown clinical intake concerning the interaction would be a burden. In some circumstances the surreptitiously captured data can be received from a pre-existing source such as a message archive, video data repository, or the like.

In the environment of **FIG. 3****,** for example, the data can be surreptitiously captured by user **335** using one or more of key(s) **361,** a mouse **362** or other pointing device, other controls **364;** or via observations by one or more instances of readers **365** (such as by optical character recognition, e.g.), cameras **366,** other sensors **368;** or the like. In the environment of **FIG. 1****,** alternatively or additionally, sensor **113** or device **110** with which a user interacts can optionally provide such data surreptitiously.

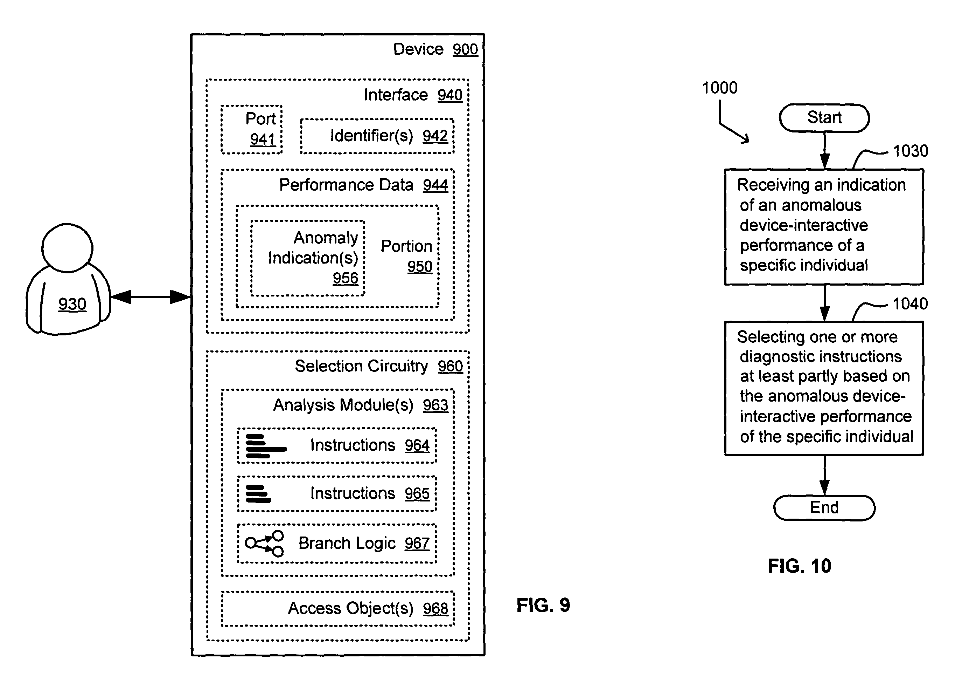

Flow **400** further includes operation **440** applying one or more data extraction criteria to the health-status-indicative data surreptitiously captured from the interaction between the device and the user (e.g. invocation module **370** and one or more instances of decision logic **381,** suitable filters **384,** or other extraction modules **333** generating distilled output **388** as described herein). In some embodiments, decision logic **381** can optionally be configured to select diagnostic instructions, for example, based on any anomalous data from the surreptitiously captured data according to flows in FIGS. **10****,** **27****,** or **28** as described in detail below. Distilled output **388** can thereby or thereafter be summarized, recorded, analyzed, or the like in various modes as described below.

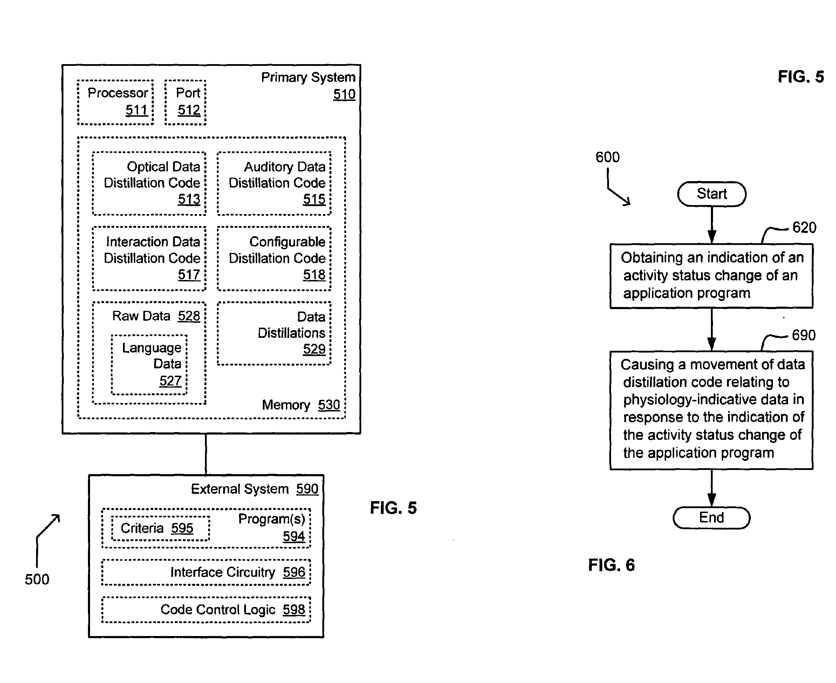

With reference now to **FIG. 5****,** shown is an example of a system that may serve as a context for introducing one or more processes and/or devices described herein. As shown system **500** can include one or more instances of primary system **510** and external system **590** operably coupled. Primary system **510** can include one or more instances of processors **511,** ports **512,** or memory **530.** Memory **530** can include (in respective "memories," e.g.) one or more instances of optical data distillation code **513,** auditory data distillation code **515,** interaction data distillation code **517,** configurable distillation code **518,** language data **527** or other raw data **528,** or data distillations **529.** External system **590** can include one or more instances of programs **594** with criteria **595,** interface circuitry **596,** or code control logic **598.**

With reference now to **FIG. 6****,** there is shown a high-level logic flow **600** of an operational process. Flow **600** includes operation **620** obtaining an indication of an activity status change of an application program (e.g. port **512** receiving or otherwise detecting an indication that one or more user programs **594** have come online, stalled, encountered an error, completed a task, or the like). If a user powers up external system **590**, for example, this can cause one or more programs **594** to be loaded or otherwise trigger such activity status changes in some configurations.

In some embodiments, "physiology-indicative" data includes any information that a knowledgeable caregiver, patient, expert system or other diagnostic agent might reasonably recognize as relevant for monitoring or understanding some attribute of test subjects or their circumstances. As exemplified herein, the physiology-indicative data can include one or more of a performance record, a measurement, or other information of potential relevance to analyzing physiological attributes of individuals such as device users, patients, workers, employers, or the like who observe or provide information to application programs. The physiology-indicative data can optionally include voice or other auditory data, pupil dilation or other optical data, device movements or other user-entered data, position information (of body parts, e.g.), or chemical or electrical sensor signals or the like to indicate data from one or more individuals. Alternatively or additionally, the physiology-indicative data can include a date or time, screen display expressions, code configuration attributes, hardware attributes, resource attributes or other information from devices and systems.

Flow **600** further includes operation **690** causing a movement of data distillation code relating to physiology-indicative data in response to the indication of the activity status change of the application program (e.g. processor **511** or code control logic **598** causing a movement of auditory data distillation code **515** into memory **530** so that it can process one or more data distillations **529** from language data **527** or other raw data **528**). In some embodiments, auditory data distillation code **515** can generate one or more data distillations **529** by removing, marking, or otherwise deemphasizing portions of an audio recording below a given decibel threshold, for example, such as a threshold of human hearing or lower. Auditory data distillation code **515** can, alternatively or additionally, present or otherwise highlight more-relevant or other anomalous auditory data such as coughing, snoring or wheezing (at least partly indicative of a respiratory disorder, e.g.); or frequent shouting, cursing, or crying (at least partly indicative of a physiological or other emotional disorder, e.g.). In some embodiments, auditory data distillation code **515** can likewise detect the device user(s) showing an increased success in responding to very soft sounds (indicative of a hearing improvement such as may result from a hearing aid or other treatment, e.g.) or exercising an increased vocabulary or performance in a memory game (at least slightly indicative of a cognitive improvement, e.g.); or the like. Those skilled in the art will recognize that speech and other patterns such as these can be recognized using commonly available algorithms, permitting implementation of a diverse array of variants of flow **600** in light of these teachings, without undue experimentation. In some embodiments, moreover, data distillations **529** can include a raw data sample, a link or other data indexing feature, or other logic for facilitating access to a portion of raw auditory data having a higher-than-nominal apparent relevance in relation to such symptoms and other indications of physiological relevance.

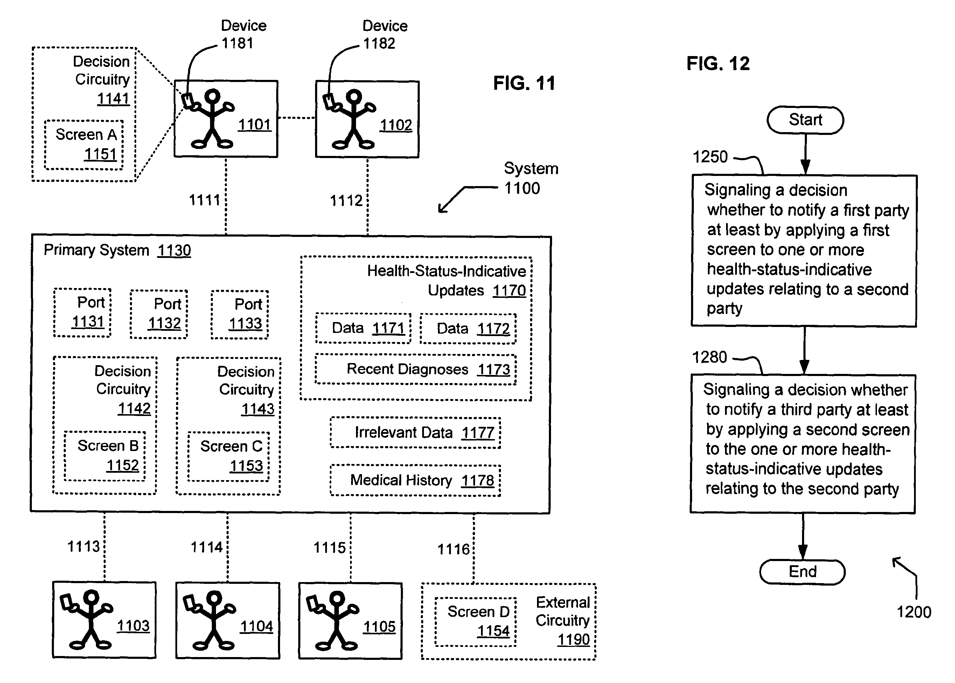

Alternatively or additionally, processor **511** can be configured to move one or more instances of optical data distillation code **513,** interaction data distillation code **517,** configurable distillation code **518** or the like into or out of memory **530** in response to the indication. Such data distillation code can (optionally) generate an evaluation or summary of some physiologically relevant data, for example, or otherwise at least partly filter out a less-relevant portion of such data or preferentially include more-relevant portions of such data. See, for example, the detailed explanations below in relation to **FIG. 11****.**

In some embodiments, the data distillation code can include a segment containing at least one instruction operable for generating, selectively retaining, or more readily accessing a portion of the data with a more-than-nominal apparent utility, relative to another portion of the data. A block of data with an apparently decreasing relevance, for example, can (optionally) be distilled by displaying or ranking a first portion first, by removing or de-emphasizing a later portion, or by sampling, highlighting, or indexing early portions at a higher sampling frequency. In some embodiments, normal or otherwise duplicative data is extracted or a diverse sampling of data is otherwise preferentially retained. Some embodiments may likewise distill frequency indicators or other evaluations signifying prevalence of an observation to indicate a higher or lower apparent utility. Alternatively or additionally, a composite evaluation of utility may account for more than one aspect of apparent relevance, such as by indicating more than one pathology of interest.

It deserves emphasis that in such embodiments as that of **FIG. 5****,** an event can occur "in response to" one or more of a prior or contemporaneous measurement, decision, transition, circumstance, or other determinant. Any such event may likewise depend upon one or more other prior, contemporaneous, or potential determinants, in various implementations as taught herein. In other words, such events can occur "in response to" one or more earlier (enabling) events as well as to a later (triggering) event in some contexts.

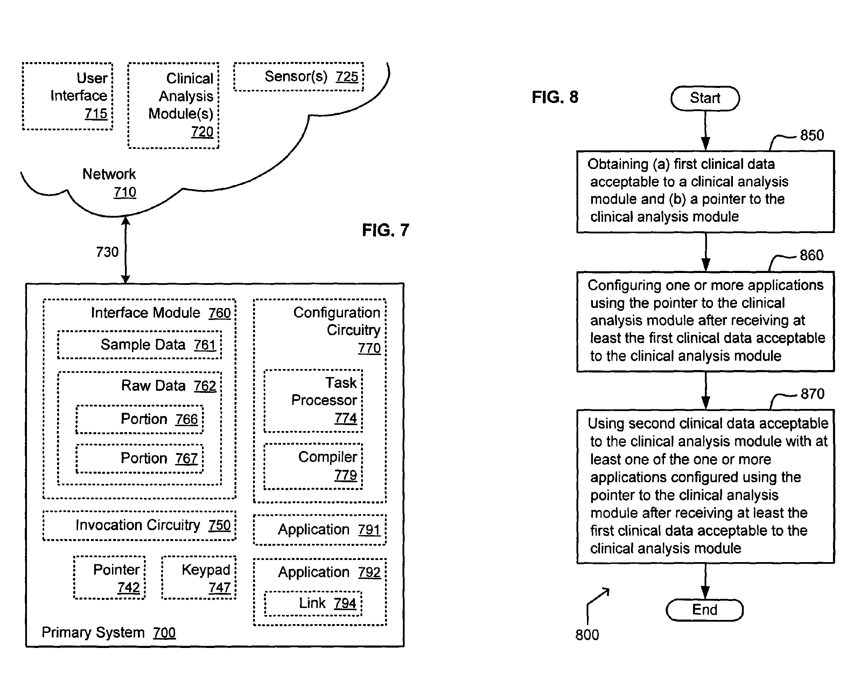

With reference now to **FIG. 7****,** shown is an example of a system that may serve as a context for introducing one or more processes and/or devices described herein. As shown primary system **700** can include one or more instances of pointers **742,** keypads **747,** invocation circuitry **750,** interface modules **760,** configuration circuitry **770,** or applications **791, 792** (optionally with links **794** as described below). Interface module **760** can include one or more instances of sample data **761,** portions **766, 767** of raw data **762,** or the like. Configuration circuitry **770** can include one or more instances of task processors **774** or compilers **779.** Primary system **700** is operable to communicate with network **710** via network interface **730;** and network **710** can include one or more instances of user interfaces **715,** clinical analysis modules **720,** or sensors **725.**

With reference now to **FIG. 8****,** there is shown a high-level logic flow **800** of an operational process. Flow **800** includes operation **850** obtaining (a) first clinical data acceptable to a clinical analysis module and (b) a pointer to the clinical analysis module (e.g. interface module **760** receiving at least sample data **761** and pointer **742).** For example, such pointers can identify or otherwise permit access to one or more clinical analysis modules **720** available remotely (e.g. via link **794)** or locally (e.g. by being downloaded or otherwise linked to application **792).** Alternatively or additionally, the data or pointer can be obtained from within primary system **700,** such as via keypad **747.** In some embodiments, the clinical data may include data (e.g. portion **767**) unacceptable to one or more of the clinical analysis modules **720.** Alternatively or additionally, such clinical data can be made acceptable to available clinical analysis modules, such as by enabling or upgrading a module at least partly based on descriptive information about sample data **761** (e.g. whether the sample data is image data, where it is from, or who it is about). In some embodiments, the data or pointer can be received across a conduit or other signal bearing medium (e.g. via network interface **730**). Optionally, interface module **760** can be configured to apply one or more criteria for determining whether sample data **761** is acceptable to the clinical analysis module(s), as described herein.

Flow **800** further includes operation **860** configuring one or more applications using the pointer to the clinical analysis module after receiving at least the first clinical data acceptable to the clinical analysis module (e.g. configuration circuitry **770** generating or adapting application **791** or application **792,** including or otherwise using one or more instances of the above-described pointers or link **794** after completing operation **850**). Configuration circuitry **770** can (optionally) include task processor **774** or compiler **779,** for example, capable of performing such configuration. In some variants, of course, operation **850** can repeat or resume after operation **860** begins, such as by user interface **715** later receiving some other such pointer(s) or some additional increment, type, or other aspect of data portion **766** (from an operator, e.g.).

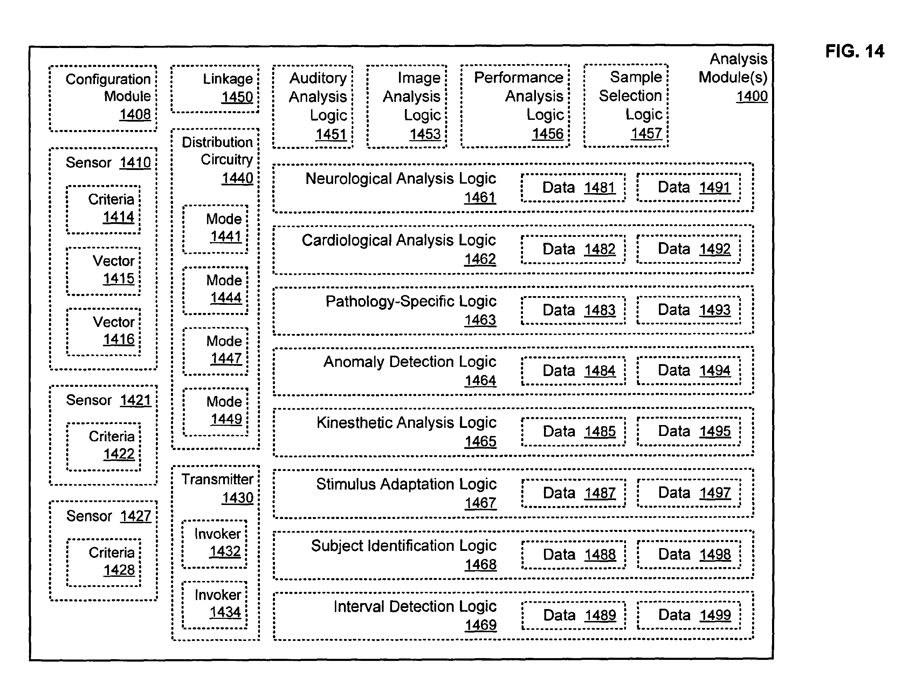

Flow **800** further includes operation **870** using second clinical data acceptable to the clinical analysis module with at least one of the one or more applications configured using the pointer to the clinical analysis module after receiving at least the first clinical data acceptable to the clinical analysis module (e.g. invocation circuitry **750** causing application **791** to use one or more of clinical analysis modules **720** upon data portion **766**). In some variants in which data portion **766** includes optical data, for example, invocation circuitry **750** can transmit or identify data portion **766** or the like to image analysis logic as described herein. See, e.g., the discussion of **FIG. 14** below. Alternatively or additionally, this can occur before or during operation **860,** such as in response to interface module **760** determining that an earlier value of pointer **742** is suitable for some or all types of clinical data arriving at interface module **760.**

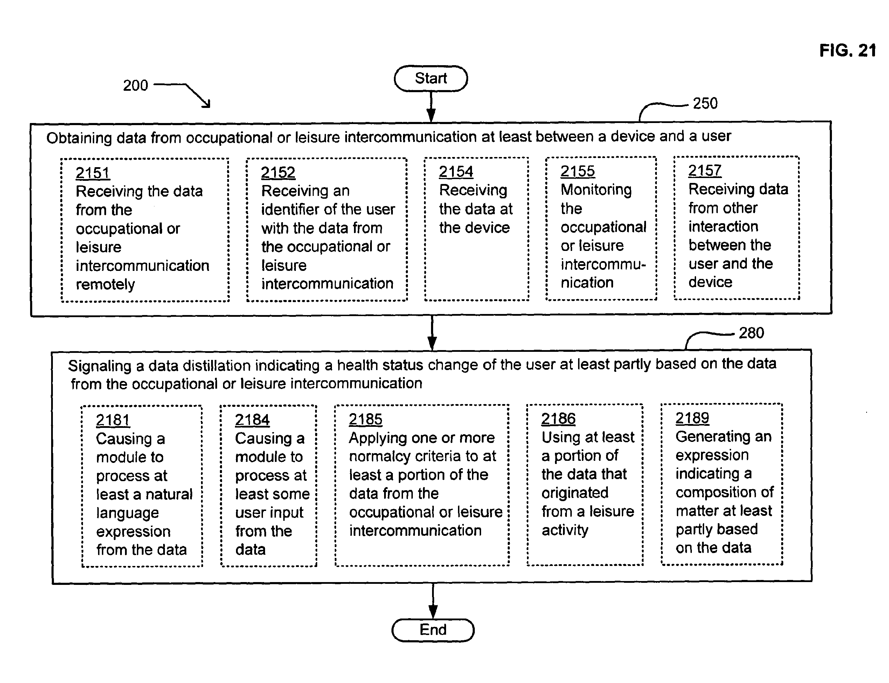

In some embodiments, flow **800** can enable getting some sample data and access to a diagnostic module (at operation **850**), installing the diagnostic module or otherwise configuring it for later inputs like the sample data (at operation **860**), and causing the diagnostic module to run on other data, such as for monitoring users (at operation **870**) as described herein. This can be particularly useful, for example, in situations in which a type or other context of raw data **762** from one or more sensor(s) **725** is not well understood. See, e.g., variants of flow 200 described below in relation to **FIGS. 21-23****.**

With reference now to **FIG. 9****,** shown is an example of a system that may serve as a context for introducing one or more processes and/or devices described herein. As shown device **900** can optionally observe or otherwise communicate with individual **930.** Device **900** can include one or more instances of interfaces **940** or selection circuitry **960.** Interface **940** can include one or more instances of port(s) **941,** identifiers **942,** or performance data **944** (such as anomaly indications **956** or other portions **950,** e.g.). Selection circuitry **960** can include one or more instances of analysis modules **963** or access objects **968.** Such an analysis module **963** can contain one or more instances of instructions **964, 965** or branch logic **967.**

With reference now to **FIG. 10****,** there is shown a high-level logic flow **1000** of an operational process. Flow **1000** includes operation **1030** receiving an indication of an anomalous device-interactive performance of a specific individual (e.g. interface **940** receiving an indication of or otherwise detecting a task or other activity that somehow deviates from normalcy in its manner, efficiency, outcome, degree, occurrence, timing, or other aspect). This can occur in the context of **FIG. 1****,** for example, by receiving raw data **182** or output **188** showing that leisure intercommunication between device **110** and user **130** indicates a level of effectiveness that differs substantially from a prior observed range of behavior for user **130,** as measured by some suitable measure of productivity or other performance. (Those skilled in the art will recognize that a "substantial" difference can reasonably comprise a deviation of at least X%, for example, with X typically in a range of 5 to 50 for contexts as described herein.)

Flow **1000** further includes operation **1040** receiving an indication of an anomalous device-interactive performance of a specific individual (e.g. selection circuitry **960** selecting special-purpose instructions **964** for analyzing data relating to specific individual **930** in a batch process using one or more anomaly indication(s) **956** or some other apparently-relevant portion **950** of performance data **944**). Such a selection can be appropriate, for example, in response to an attribute of some performance data in response to an activity status change of the user's application program (according to variants of flow **600** described herein, for example), in response to data surreptitiously captured (according to variants of flow 400 described herein, for example), or the like.

In some embodiments, "diagnostic" instructions can include special-purpose device instruction sequences directly or automatically enabling or otherwise able to cause an analysis of one or more user data or the like as described herein. Alternatively or additionally, a diagnostic instruction can be an oral or written instruction given to a patient or other caregiver, for example, prompting an action or omission primarily to facilitate a diagnostic test. Moreover, some "diagnostic" instructions can include software of which at least a portion prompts or guides such an action or omission.

With reference now to **FIG. 11****,** shown is an example of a system that may serve as a context for introducing one or more processes and/or devices described herein. As shown system **1100** includes at least one primary system **1130** operable to communicate with several parties **1101, 1102, 1103, 1104, 1105** via respective linkages **1111, 1112, 1113, 1114, 1115.** For example, linkage **1111** can be implemented via device **1181,** which can also include screen A **1151** or other decision circuitry **1141.** As shown, device **1182** can optionally observe or otherwise communicate with one or more of party **1101** (directly as shown, e.g.), party **1102,** or primary system **1130.** Primary system 1130 can include one or more instances of ports **1131, 1132, 1133,** screen B **1152** or other decision circuitry **1142,** screen C **1153** or other decision circuitry **1143,** health-status-indicative updates **1170,** irrelevant data **1177,** medical history **1178,** or the like. Health-statu-indicative updates **1170** can include one or more instances of data **1171, 1172** or recent diagnoses **1173.** Primary system can also likewise be operable to access screen D **1154** or other external circuitry **1190** via linkage **1116.** In some variants, one or more of the linkages **1111-1116** shown are wireless or otherwise direct (e.g. via a passive-media signal path).

With reference now to **FIG. 12****,** there is shown a high-level logic flow **1200** of an operational process. Flow **1200** includes operation **1250** signaling a decision whether to notify a first party at least by applying a first screen to one or more health-status-indicative updates relating to a second party (e.g. some of decision circuitry **1141, 1142, 1143** implementing or otherwise signaling a decision in response to one or more screens **1151-1153** applied to update data relating to the "second" party, of whether or when to notify the "first" party). The "second" party in this context can include one or more subjects of observation such as parties **1101, 1102.** The "first" party in this context can include one or more other parties **1101-1105** to be notified. The update data can include specific health-status-indicative updates **1170** as well as other information such as irrelevant data **1177** or medical history **1178** relating to such a patient. Such health-status-indicative updates **1170** can be configured or otherwise designated for distillation as described in variants of flows **200** or **600** described below, for example, such as by retaining apparently-more-relevant data **1171** or by filtering out apparently-less-relevant data **1172.**

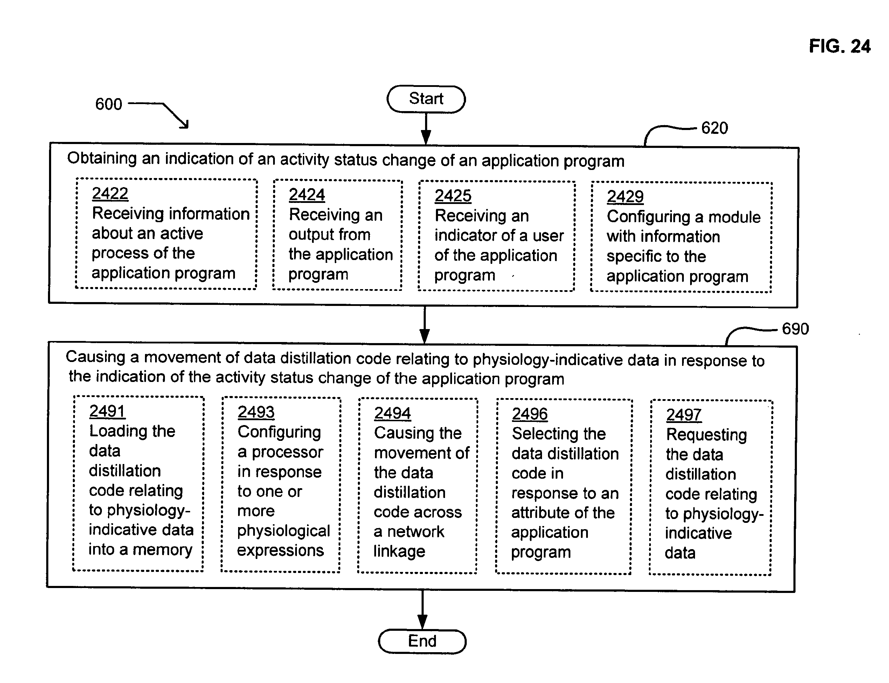

Flow **1200** further includes operation **1280** signaling a decision whether to notify a third party at least by applying a second screen to the one or more health-status-indicative updates relating to the second party (e.g. other decision circuitry **1142, 1143** or external circuitry **1144** signaling a decision resulting from applying one or more other screens **1152-1154** at least to some of the update information relating to the "second" party). The decision can specify how, when, or whether the "third" party (e.g. parties **1101, 1105)** is or was notified. The content of such notifications can, for example, can include one or more distillations relating to health-status-indicative data or other information as described herein. See, e.g., **FIGS. 24-29** and accompanying descriptions below.

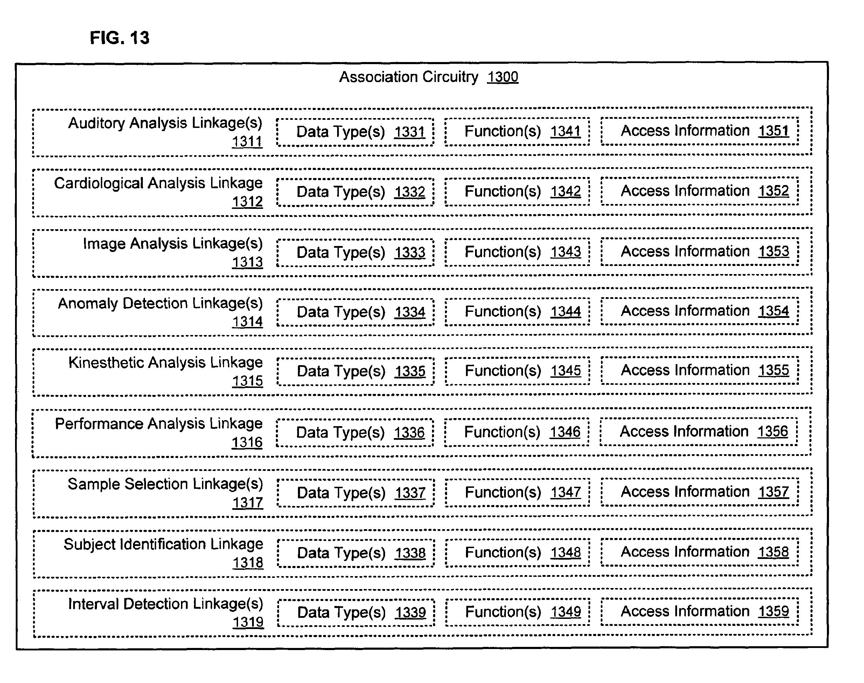

With reference now to **FIG. 13****,** shown is an example of circuitry that may serve as a context for introducing one or more processes and/or devices described herein. As shown association circuitry **1300** can (optionally) include one or more instances of auditory analysis linkages **1311** linking with one or more instances of data types **1331,** functions **1341,** or access information **1351** described herein in relation to auditory analysis or the like. Association circuitry **1300** can likewise include one or more instances of cardiological analysis linkages **1312** linking with one or more instances of data types **1332,** functions **1342,** or access information **1352** described herein in relation to cardiological analysis or the like. Association circuitry **1300** can likewise include one or more instances of image analysis linkages **1313** linking with one or more instances of data types **1333,** functions **1343,** or access information **1353** described herein in relation to image analysis, manipulation, or the like. Association circuitry **1300** can likewise include one or more instances of anomaly detection linkages **1314** linking with one or more instances of data types **1334,** functions **1344,** or access information **1354** described herein in relation to anomaly detection, analysis, or the like. Association circuitry **1300** can likewise include one or more instances of kinesthetic analysis linkages **1315** linking with one or more instances of data types **1335,** functions **1345,** or access information **1355** described herein in relation to kinesthetic analysis or the like. Association circuitry **1300** can likewise include one or more instances of performance analysis linkages **1316** linking with one or more instances of data types **1336,** functions **1346,** or access information **1356** described herein in relation to performance analysis or the like. Association circuitry **1300** can likewise include one or more instances of sample selection linkages **1317** linking with one or more instances of data types **1337,** functions **1347,** or access information **1357** described herein in relation to sample selection, sample analysis, or the like. Association circuitry **1300** can likewise include one or more instances of subject identification linkages **1318** linking with one or more instances of data types **1338,** functions **1348,** or access information **1358** described herein in relation to subject identification, comparative analysis, group inclusion, or the like. Association circuitry **1300** can likewise include one or more instances of interval detection linkages **1319** linking with one or more instances of data types **1339,** functions **1349,** or access information **1359** described herein in relation to interval detection, timing analysis, or the like. Any of such linkages **1311-1319** can, of course, take any of many forms discernable by teachings herein or known in the art, such as by pointers, names, protocols, predetermined structures, hard wiring or coding, or the like. Some varieties or instances of association circuitry can be implemented, for example, in device **110** or primary system **100** of **FIG. 1****,** in handheld or other devices **300** or external module **380** of **FIG. 3****,** in primary system **510** or external system **590** of **FIG. 5****,** in clinical analysis modules **720** or primary system **700** of **FIG. 7****,** in device **900** of **FIG. 9****,** in primary system **1130** or devices **1181, 1182** of **FIG. 11****,** within other modules or media as described herein, or for handling other data as described herein.

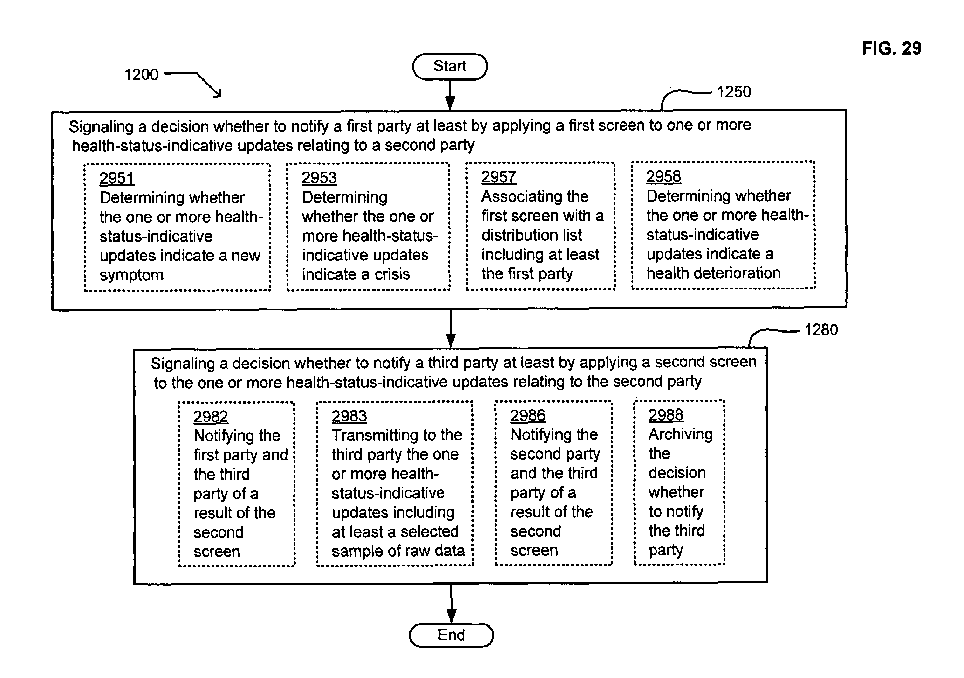

With reference now to **FIG. 14****,** shown is an example of a system that may serve as a context for introducing one or more processes and/or devices described herein. As shown the system can include analysis module(s) **1400** comprising one or more instances of configuration modules **1408,** sensors **1410, 1421, 1428,** linkages **1450,** distribution circuitry **1440,** or invokers **1432, 1434** or other transmitters **1430.** Sensor **1410** can include one or more criteria such as those described below relating to vectors **1415, 1416** as described below in relation to **FIG. 29****.** Sensors **1421, 1427** can (optionally) each respectively implement one or more criteria **1422, 1428.** Linkage **1450** can implement some or all of association circuitry **1300,** in some embodiments. Distribution logic **1440** can likewise implement one or more instances of modes **1441, 1444, 1447, 1449** as described below, for example, in relation to **FIG. 29****.**

Analysis module(s) **1400** can further include one or more instances of auditory analysis logic **1451,** image analysis logic **1453,** performance analysis logic **1456,** sample selection logic **1457,** neurological analysis logic **1461,** cardiological analysis logic **1462,** pathology-specific logic **1463,** anomaly detection logic **1464,** kinesthetic analysis logic **1465,** stimulus adaptation logic **1467,** subject identification logic **1468,** interval detection logic **1469,** or the like. At various times and in various embodiments, analysis module(s) **1400** can likewise obtain one or more instances of data **1481, 1482, 1483, 1484, 1485, 1487, 1488, 1489** or data **1491, 1492, 1493, 1494, 1495, 1497, 1498, 1499** respectively as shown. It deserves emphasis that many of these data items can be generated by or otherwise relate to other logic modules than that shown explicitly in **FIG. 14****.** Data **1498** of subject identification logic **1468,** for example, can relate just as strongly with one or more of image analysis logic **1456,** sample selection logic **1457,** neurological analysis logic **1461,** or pathology-specific logic **1463.**

In some variants of analysis module(s) **1400,** some or all of logic **1451, 1453, 1456, 1457, 1461, 1462, 1463, 1464, 1465, 1467, 1468, 1469** can (optionally) be configured automatically, without any end-user input. Alternatively or additionally, some or all of logic **1451, 1453, 1456, 1457, 1461, 1462, 1463, 1464, 1465, 1467, 1468, 1469** can be configured in response to a user-specific goal. Neurological analysis logic **1461,** for example, can be configured in response to a request to check a user's reaction time against a nominal standard such as the user's own baseline or that of a population that includes the user.

Alternatively or additionally, some or all of logic **1451, 1453, 1456, 1457, 1461, 1462, 1463, 1464, 1465, 1467, 1468, 1469** can (optionally) be configured to monitor a user or interaction passively-from outside an application or other user function(s) with which a user interacts, for example. In some implementations, such logic can be merely responsive, such as by configuring auditory analysis logic **1451** to be effective for obtaining or retaining a record of vomiting, flatulence, smoking, or other distinguishable audible phenomena susceptible of indicative recordation or other detection sufficient to provide data relating to an individual's health using available system resources. Alternatively or additionally, some or all of logic **1451, 1453, 1456, 1457, 1461, 1462, 1463, 1464, 1465, 1467, 1468, 1469** can be configured as a part of the application or other user function(s) with which the user interacts-such as by adapting stimulus adaptation logic **1467** so that the user can see or hear different stimuli.

Alternatively or additionally, pathology-specific logic **1463** can be chosen or configured to detect, test, infer, predict, or diagnose, and/or to confirm, refute or otherwise test a hypothesis of interest for one or more specific individuals. Such analysis can be performed in relation to participants in a virtual reality environment, for example, measuring reaction times, color perceptions, visual fields, losses in performance when switching tasks, ability to process multiple tasks, short term memory, long term memory, medication side effects, medication efficacy, correlates of medication compliance, or the like as data **1483, 1493.**

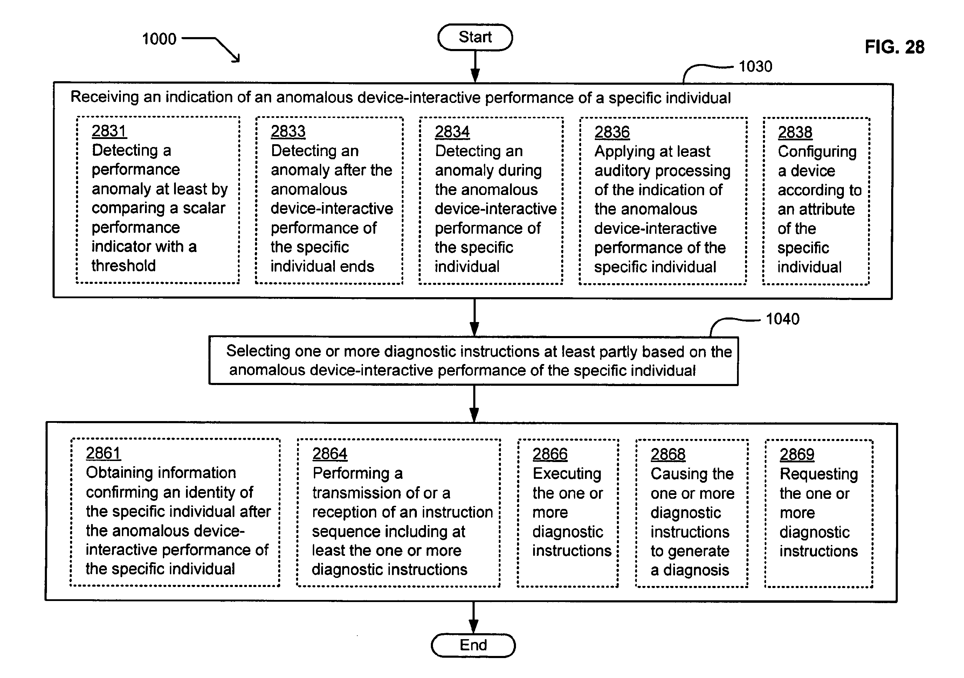

Alternatively or additionally, measurement data **1489** or other inference data **1499** from interval detection logic **1469** can be used by one or more others of logic **1451, 1453, 1456, 1457, 1461, 1462, 1463, 1464, 1465, 1467, 1468.** Subject identification logic **1468** can respond to a drastic reaction time improvement, for example, by inferring a likelihood that a new individual is now using a given interface. See **FIG. 28****.**

Alternatively or additionally, anomaly detection logic **1464** can detect normalcy-indicative data **1484** in one, two, or several aspects: reaction time, user effectiveness, user appearance, heart rate or other bioinformatic data, user location, or the like. In some variants, anomaly detection logic **1464** can be configured to analyze raw input data (from data **1484,** e.g.) and use it for detecting whether an abnormal level of ambient light or noise or temperature or other attributes exist in a vicinity of an input device (keyboard or other sensor, e.g.). Alternatively or additionally, anomaly detection logic **1464** can be configured to detect anomalies in a correlation or other relationship among two or more measured parameters: a user's apparent capacities or bioinformatic data, times of day, environmental attributes, inputs from a third party (nurse or parent, e.g.), comparisons with historical or other benchmark data, or the like.

Alternatively or additionally, some or all data **1481, 1482, 1483, 1484, 1485, 1487, 1488, 1489** or data **1491, 1492, 1493, 1494, 1495, 1497, 1498, 1499** can give rise to one or more of broadcasting to multiple parties or selective notifications (see **FIG. 12****,** e.g.), sampling or aggregation, real-time processing or other distillation, storage or other follow-up actions, or the like. Some varieties or instances of analysis modules **1400** can be implemented, for example, in device **110** or primary system **100** of **FIG. 1****,** in primary system **510** or external system **590** of **FIG. 5****,** in clinical analysis modules **720** or primary system **700** of **FIG. 7****,** in device **900** of **FIG. 9****,** in primary system **1130** or devices **1181, 1182** of **FIG. 11****,** within other modules or media as described herein, as a stand-alone system, and/or for handling other data as described herein.

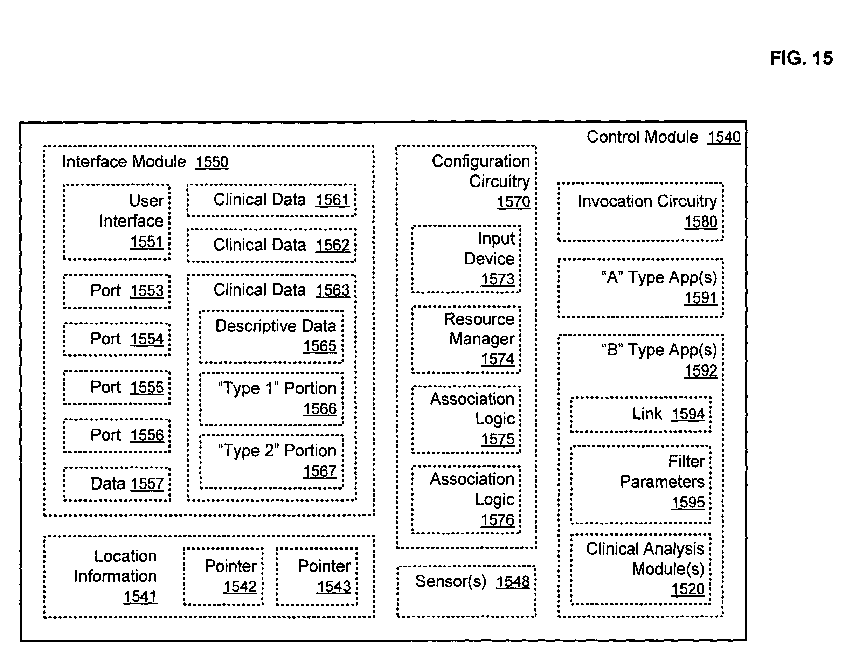

With reference now to **FIG. 15****,** shown is an example of a system that may serve as a context for introducing one or more processes and/or devices described herein. As shown the system includes at least control module **1540** including one or more instances of location information **1541** or pointers **1542, 1543,** sensors **1548,** interface modules **1550,** configuration circuitry **1570,** invocation circuitry **1580,** "A" type applications **1591,** or "B" type applications **1592.** Interface module **1550** can, for example, include one or more instances of user interfaces **1551;** ports **1553, 1554, 1555, 1556, 1557;** or clinical data **1561, 1562, 1563.** Clinical data **1563** can include one or more instances of descriptive data **1565,** "Type 1" portions **1575,** or "Type 2" portions **1567.** Configuration circuitry **1570** can include one or more instances of input devices **1573,** resource managers **1574,** or association logic **1575, 1576.** "B" type applications **1592** can include one or more instances of links **1594,** filter parameters **1595,** or clinical analysis modules **1520** such as those of **FIG. 14****.** Some varieties or instances of control module **1500** can be implemented, for example, in device **110** or primary system **100** of **FIG. 1****,** in primary system **510** or external system **590** of **FIG. 5****,** in clinical analysis modules **720** or primary system **700** of **FIG. 7****,** in device **900** of **FIG. 9****,** within other modules or media as described herein, or for handling other data as described herein.

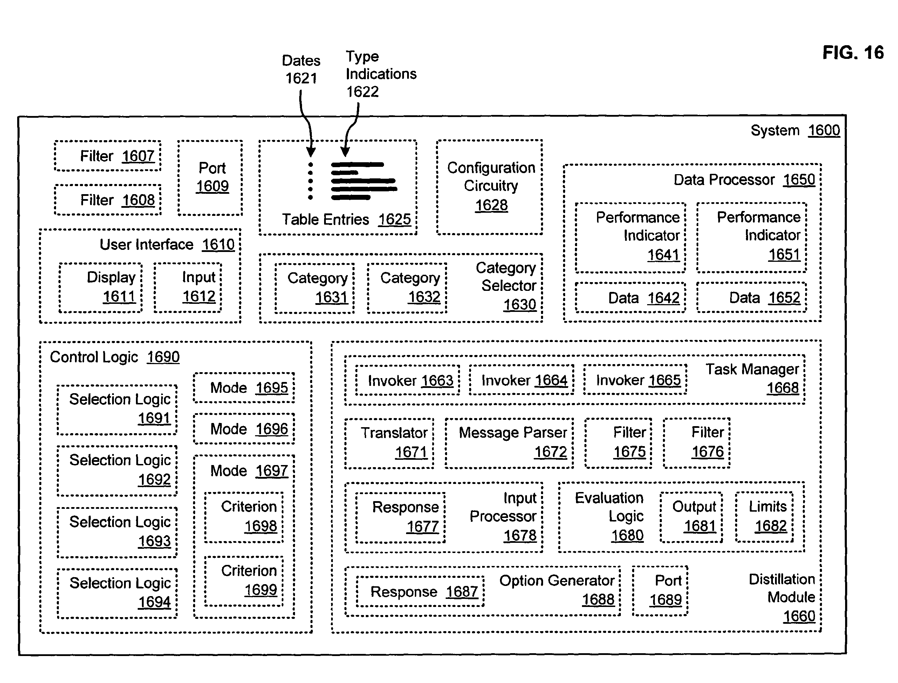

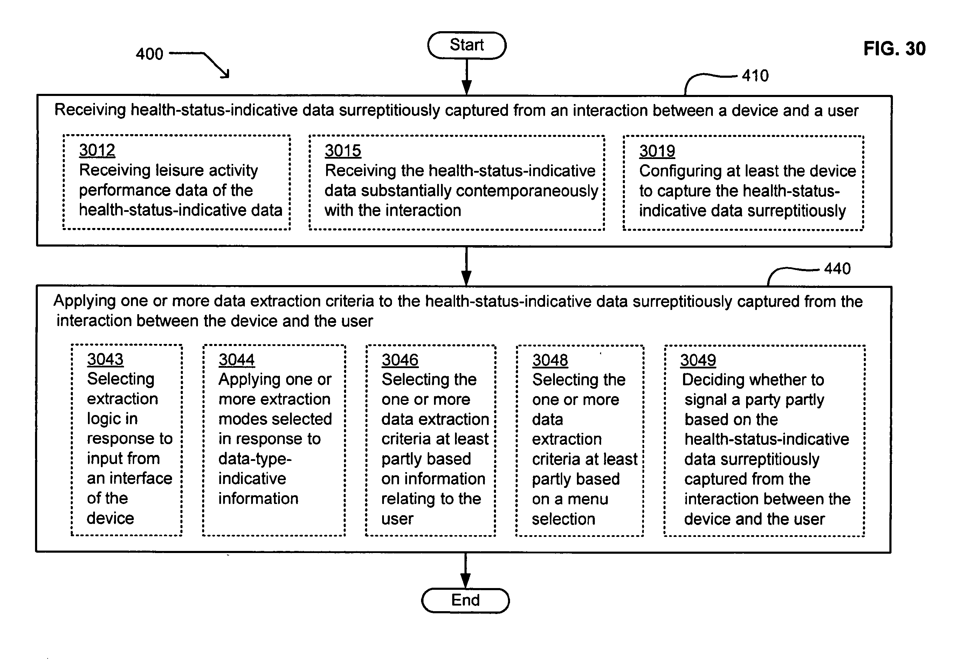

With reference now to **FIG. 16****,** shown is an example of a system that may serve as a context for introducing one or more processes and/or devices described herein. As shown system **1600** can include one or more instances of filters **1607, 1608,** ports **1609,** user interfaces **1610,** table entries **1625** comprising at least dates **1621** and type indications **1622,** configuration circuitry **1628,** category selectors **1630** operable for selecting one or more categories **1631, 1632,** data processors **1650,** distillation modules **1660,** or control logic **1690.** Data processor **1650** can handle or otherwise include one or more instances of performance indicators **1641, 1651** or data **1642, 1652.** Control logic **1690** can (optionally) include one or more instances of selection logic **1691, 1692, 1693, 1694** or one or more modes **1695, 1696, 1697.** At least mode **1697** relates to one or more criteria **1698, 1699** as described below with reference to **FIG. 30****.**

As shown, distillation module **1660** can (optionally) include one or more instances of invokers **1663, 1664, 1665** in one or more task managers **1668,** translators **1671,** message parsers **1672,** filters **1675, 1676,** input processors **1678,** evaluation logic **1680,** option generators **1688,** or ports **1689.** As described below with reference to **FIG. 21****,** input processor **1678** can optionally obtain one or more responses **1677** as can option generator **1688** obtain one or more responses **1688.** Likewise evaluation logic **1680** can obtain one or more instances of outputs **1681** or limits **1682.** Some varieties or instances of system **1600** can be implemented, for example, in device **110** or primary system **100** of **FIG. 1****,** in handheld or other devices **300** or external module **380** of **FIG. 3****,** in clinical analysis modules **720** or primary system **700** of **FIG. 7****,** in device **900** of **FIG. 9****,** in primary system **1130** or devices **1181, 1182** of **FIG. 11****,** within other modules or media as described herein, or for handling other data as described herein.

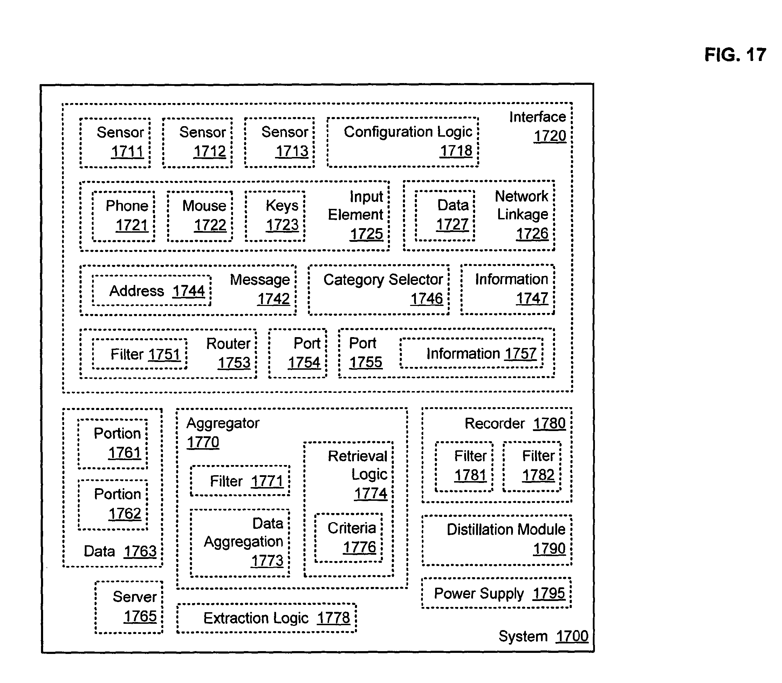

With reference now to **FIG. 17****,** shown is an example of a system that may serve as a context for introducing one or more processes and/or devices described herein. As shown system **1700** can include one or more instances of interfaces **1720,** portions **1761, 1762** of data **1763,** servers **1765,** aggregators **1770,** evaluation logic **1778,** recorders **1780,** distillation modules **1790,** or power supplies **1795.** Recorder **1780** can, for example, include one or more instances of filters **1781, 1782.** Aggregator **1770** can include one or more instances of filters **1771,** data aggregations **1773,** or retrieval logic **1774** (optionally with one or more criteria **1776**). Interface **1720** can include one or more instances of sensors **1711, 1712, 1713;** configuration logic **1718;** various types of phones **1721,** mice **1722,** keys **1723,** or other input elements **1725;** network linkages **1726** for handling data **1727;** messages **1744** (optionally with one or more addresses **1744** or the like); category selectors **1746;** information **1747;** routers **1753** (optionally with one or more filters **1751**); or ports **1754, 1755** (optionally with other information **1757**). Some varieties or instances of system **1700** can be implemented, for example, in device **110** or primary system **100** of **FIG. 1****,** in handheld or other devices **300** or external module **380** of **FIG. 3****,** in clinical analysis modules **720** or primary system **700** of **FIG. 7****,** in device **900** of **FIG. 9****,** in primary system **1130** or devices **1181, 1182** of **FIG. 11****,** within other modules or media as described herein, or for handling other data as described herein.

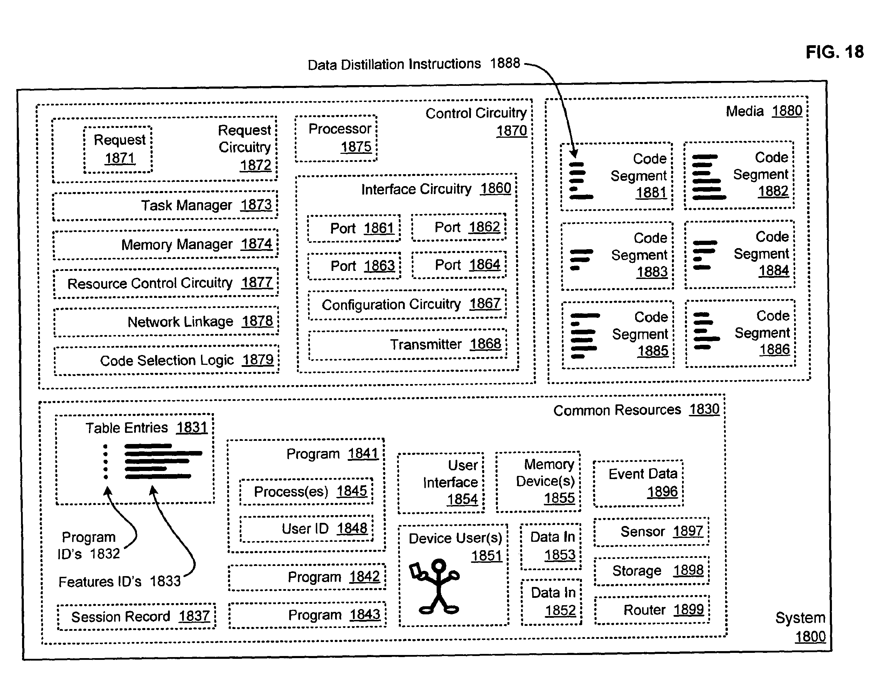

With reference now to **FIG. 18****,** shown is an example of a system that may serve as a context for introducing one or more processes and/or devices described herein. As shown system **1800** can (optionally) include one or more instances of control circuitry **1870,** media **1880,** or common resources **1830.** Control circuitry **1870** can include one or more instances of interface circuitry **1860,** request circuitry **1872** for handling requests **1871,** task managers **1873,** memory managers **1874,** processors **1875,** resource control circuitry **1877** (including or otherwise accessing resources), network linkages **1878,** code selection logic **1879.** Interface circuitry **1860** can include one or more of ports **1861, 1862, 1863, 1864;** configuration circuitry **1867;** or transmitters **1868.** One or more media **1880** can each include one or more instances of data distillation instructions **1888** or the like in instruction sequences or other code segments **1881, 1882, 1883, 1884, 1885, 1886.** Common resources **1830** can include one or more instances of table entries **1831** comprising one or more program identifiers linked with at least feature identifiers **1833** (see **FIG. 24****);** session records **1837;** programs **1841, 1842, 1843** optionally with processes **1845** linking with user identifier(s) **1848;** user interfaces **1854** and device users **1851;** memory devices **1855;** data in **1852, 1853;** event data **1896;** sensors **1897;** storage **1898;** or routers **1899.** Some varieties or instances of system **1800** can be implemented, for example, in device **110** or primary system **100** of **FIG. 1****,** in primary system **510** or external system **590** of **FIG. 5****,** in clinical analysis modules **720** or primary system **700** of **FIG. 7****,** in device **900** of **FIG. 9****,** within other modules or media as described herein, as a hand-held or other portable system, or for handling other data as described herein.

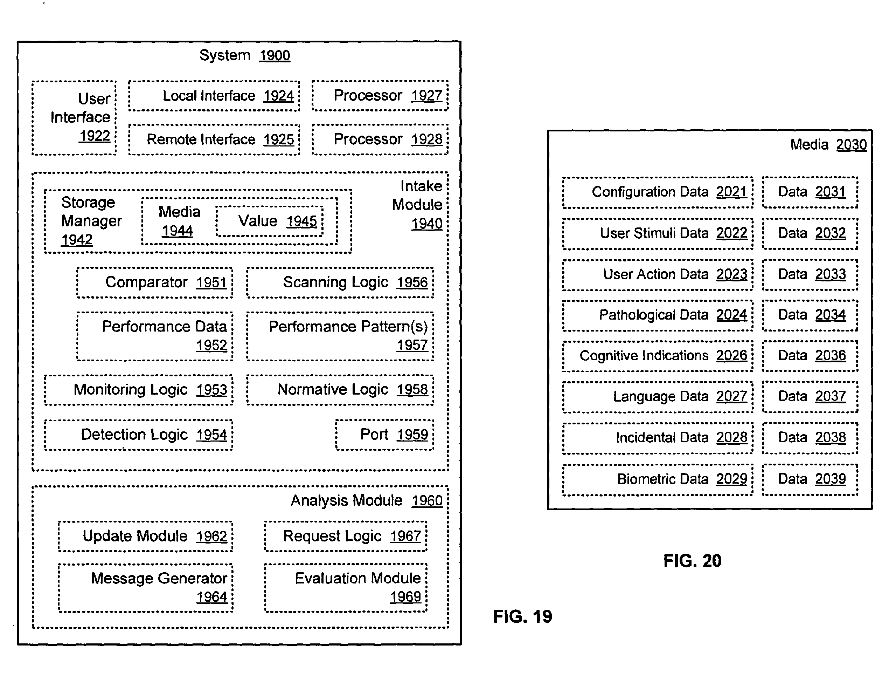

With reference now to **FIG. 19****,** shown is an example of a system that may serve as a context for introducing one or more processes and/or devices described herein. As shown system **1900** can include one or more instances of interfaces **1922, 1924, 1925;** processors **1927, 1928;** intake modules **1940;** or analysis modules **1940.** As shown, intake module **1940** can include one or more instances of storage manager **1942** (with values **1945** in one or more media **1944,** e.g.), comparators **1951,** performance data **1952,** monitoring logic 1953, detection logic **1954,** scanning logic **1956,** performance patterns **1957,** normative logic **1958,** or ports **1959.** Analysis logic can likewise (optionally) include one or more instances of update modules **1962,** message generators **1964,** request logic **1967,** or evaluation modules **1969.** Some varieties or instances of system **1900** can be implemented, for example, in device **110** or primary system **100** of **FIG. 1****,** in primary system **510** or external system **590** of **FIG. 5****,** in clinical analysis modules **720** or primary system **700** of **FIG. 7****,** in device **900** of **FIG. 9****,** in primary system **1130** or devices **1181, 1182** of **FIG. 11****,** within other modules or media as described herein, or for handling other data as described herein.

With reference now to **FIG. 20****,** shown is an example of one or more tangible and/or physical media that may serve as a context for introducing one or more processes and/or devices described herein. As shown media **2030** can include one or more instances of configuration data **2021,** user stimuli data **2022,** user action data **2023,** pathological data **2024,** cognitive indications **2026,** language data **2027,** incidental data **2028,** biometric data **2029,** or other data **2031, 2032, 2033, 2034, 2035, 2036, 2037, 2038, 2039** as described below. Some varieties or instances of media **2030** can be implemented, for example, in device **110** or primary system **100** of **FIG. 1****,** in primary system **510** or external system **590** of **FIG. 5****,** in clinical analysis modules **720** or primary system **700** of **FIG. 7****,** in device **900** of **FIG. 9****,** in primary system **1130** or devices **1181, 1182** of **FIG. 11****,** or for handling other modules or data as described herein.

With reference now to **FIG. 21****,** there are shown several variants of the flow **200** of **FIG. 2****.** Operation **250** obtaining data from occupational or leisure intercommunication at least between a device and a user may include one or more of the following operations: **2151, 2152, 2154, 2155,** or **2157.** Operation **280** signaling a data distillation indicating a health status change of the user at least partly based on the data from the occupational or leisure intercommunication may include one or more of the following operations: **2181, 2184, 2185, 2186,** or **2189.**

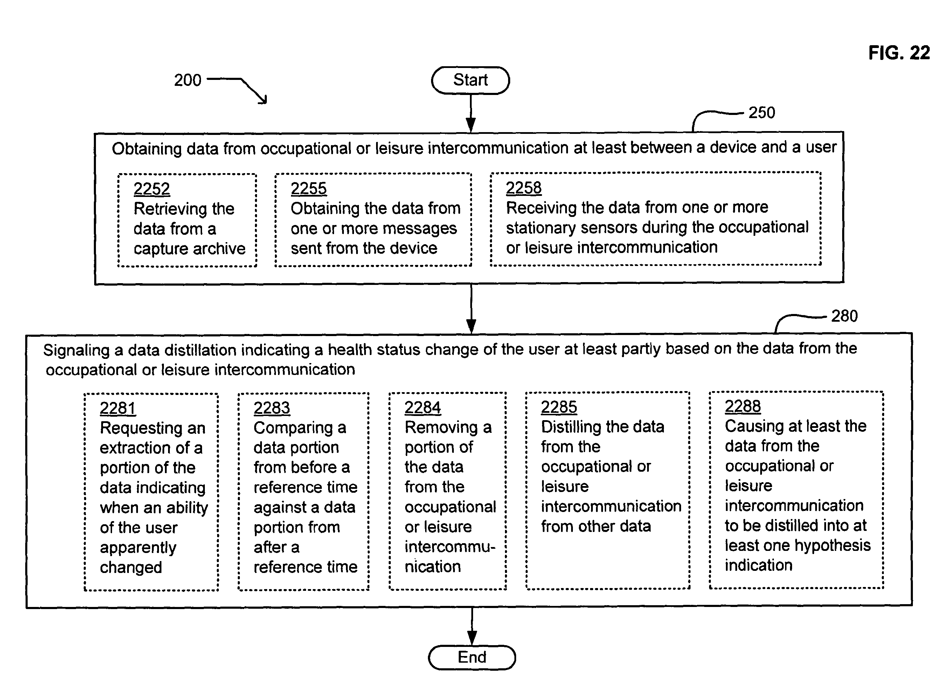

Operation **2151** describes receiving the data from the occupational or leisure intercommunication remotely (e.g. network linkage **171** receiving portions or other indications of intercommunication **135** between user **130** and device **110** as raw data **182**). This can occur, for example, in embodiments in which interface module **170** performs operation **250** and in which distillation module **180** performs operation **280.** Alternatively or additionally, instances of operation **250** can be performed by embodiments of leisure interfaces **111,** occupational interfaces **112,** or sensors **113.** Likewise some variants of operation **280** can be performed by some implementations of leisure interface **111,** occupational interface **112,** or other modes of signaling a distillation as described herein. **See** **FIGS. 22-30****.**

Operation **2152** describes receiving an identifier of the user with the data from the occupational or leisure intercommunication (e.g. server **1765** receiving a text message **1742** or the like containing the sender or recipients' address **1744**). A user reading messages or composing sentences steadily and consistently more slowly over a course of months or years may be manifesting a vision, cognitive, or psychological health status change worthy of investigation, for example. In some variants described herein, data can be aggregated from activities of more than one user and compared or otherwise distilled.

Operation **2154** describes receiving the data at the device (e.g. microphone or other phone **1721,** mouse **1722,** keys **1723,** or other input elements **1725** of device **110** receiving user input as a part of the intercommunication). This can occur, for example, in embodiments in which device **110** include one or more instances of system **1700.** In a context in which data processing retention is burdensome, in some embodiments one side of the intercommunication (from device **110** to user 130, e.g.) can optionally be ignored.

Operation **2155** describes monitoring the occupational or leisure intercommunication (e.g. one or more of sensors **1712, 1713** detecting intercommunication **135** with occupational interface **112** or some other device in a workplace). This can occur, for example, in embodiments in which device **110** includes one or more instances of systems **1700,** with or without any implementation of primary system **100.**

Operation **2157** describes receiving data from other interaction between the user and the device (e.g. port **1755** receiving global positioning or other information **1757** from the device, such as to indicate that the user apparently moved the device or otherwise changed its status other than by "intercommunication" with the device). In some contexts, such data from other interaction or other intercommunications can provide contextual information helpful to a data analyst trying to diagnose the apparent health status change or otherwise interpret a record of the intercommunication.

Operation **2181** describes causing a module to process at least a natural language expression from the data (e.g. invoker **1663** causing message parser **1672** to determine whether user **130** is saying something health related). Alternatively or additionally, optical character recognition can be invoked for counting typos or otherwise analyzing text data from a user objectively. Speech recognition can likewise be invoked for detecting slurred speech, long pauses, or other objectively detectable phenomena that can indicate a drug overdose or other health status change of user **130.** Such variants can optionally be used with translator **1671** (Spanish to English, e.g.) operable for making a user's speech accessible to a clinical analysis module or caregiver in some embodiments.

Operation **2184** describes causing a module to process at least some user input from the data (e.g. invoker **1664** causing input processor **1678** to interpret keystrokes, sounds, gestures, or the like as an affirmative or negative response **1677**). Alternatively or additionally, response **1677** can include one or more instances of location information (via mouse **1722,** e.g.), user selections, responses to diagnostic stimuli (as data **1487** from stimulus adaptation logic **1467**), or the like.

Operation **2185** describes applying one or more normalcy criteria to at least a portion of the data from the occupational or leisure intercommunication (e.g. evaluation logic **1680** comparing a game score or other application output **1681** against one or more normal minimum or maximum limits **1682**). One or more such limits can be defined for a particular user or group of users, for example, dependent upon age, experience at the task, past performance, or the like. Occupational output for a typist can include a typing rate or error rate against a normal minimum or maximum for that typist or for some population of similar typists.

Operation **2186** describes using at least a portion of the data that originated from a leisure activity (e.g. one or more analysis module(s) **1400** using leisure-type intercommunication data such as that of a user chatting or playing a computer game). In some variants of operation **2086,** the same or other module(s) can also analyze occupational-type intercommunication data.

Operation **2189** describes generating an expression indicating a composition of matter at least partly based on the data (e.g. option generator **1688** indicating one or more nutraceuticals or other products sometimes used for the apparent health status change). A user showing signs of insomnia, for example, may trigger a response **1687** including chamomile, Lunesta®, ear plugs, or memory foam products. The list may be sent to the user, to a caregiver, or to other interested parties such as advertisers. In some variants, option generator can merely request such options from a remote source and later provide options from whatever response **1687** is received.

With reference now to **FIG. 22****,** there are shown several variants of the flow **200** of **FIG. 2** or **21****.** Operation **250** obtaining data from occupational or leisure intercommunication at least between a device and a user may include one or more of the following operations: **2252, 2255,** or **2258.** Operation **280** signaling a data distillation indicating a health status change of the user at least partly based on the data from the occupational or leisure intercommunication may include one or more of the following operations: **2281, 2283, 2284, 2285,** or **2288.**

Operation **2252** describes retrieving the data from a capture archive (e.g. retrieval logic **1774** applying search criteria **1776** to extract location, identity, timing, performance, or the like from data aggregation **1773**). This can occur, for example, in embodiments in which one or more instances of aggregators **1770** or interfaces **1610, 1720** perform operation **250,** in which one or more instances of distillation modules **1660, 1790** perform operation **280,** or in which system **1700** is implemented as a stand-alone system or within an embodiment of system **1600.**

Operation **2255** describes obtaining the data from one or more messages sent from the device (e.g. filter **1751** detecting medical terms, stress-indicative terms, or the like in voice or text data **1763**). In some embodiments, filter **1751** can be configured to search for a pattern that contains natural language terms or other indications of user excitement or distress, anomalies of volume or pace, user-affecting stimuli such as messages from third parties (or from occupational or leisure software), or the like. Alternatively or additionally, filter **1751** can be configured to include some other data in a vicinity of the pattern, or only those packets or the like that contain the pattern.

Operation **2258** describes receiving the data from one or more stationary sensors during the occupational or leisure intercommunication (e.g. network linkage **1726** receiving data **1727** generated by one or more peripheral devices, ad hoc network nodes, or the other stationary elements containing a sensor or other input). In some variants, the device with which the user interacts can include one or more such stationary sensors. For example, one or more instances of sensors **113** within a vicinity of user **130** (e.g. site **120**) can security cameras, microphones, touchscreens, or the like.

Operation **2281** describes requesting an extraction of a portion of the data indicating when an ability of the user apparently changed (e.g. invoker **1665** triggering a remote resource to estimate when or whether the user started to exhibit a symptom based on an available record). The remote resource may be a medical diagnostic program, for example, or a physician with a specialty. The available record can include any communication-indicative or other data potentially indicative of a health status change. The request can be a general request or may otherwise refer to matters other than ability change timing. In some instances a response might indicate that the ability has apparently not changed, or it may only narrow down the interval during which the change apparently occurred.

Operation **2283** describes comparing a data portion from before a reference time against a data portion from after a reference time (e.g. data processor **1650** computing performance indicators **1641, 1651** from data **1642, 1652** for respective periods before and after a regimen change or the like). This can be used as a basic comparator (to indicate an apparent 10% loss in performance, e.g.), for hypothesis testing, or for applying other mathematical models to the data **1642, 1652.** The reference time can be provided at a user interface, for example, or can be derived from data **1642, 1652** or other medical history information.

Operation **2284** describes removing a portion of the data from the occupational or leisure intercommunication (e.g. filter **1675** removing an irrelevant or otherwise unwanted portion of any of data **1481-1499** of **FIG. 14**). This can occur, for example, in embodiments in which one or more interfaces **1610, 1720** perform operation **250,** in which distillation module **1660** performs operation **280,** and in which distillation module **1660** accesses or otherwise implements analysis module(s) **1400.** Such distillations can be performed by selectively retaining portions near an event or time of interest, for example, or by removing noise or the like.

Operation **2285** describes distilling the data from the occupational or leisure intercommunication from other data (e.g. filter **1676** removing data relating to other users or devices, data from other types of interactions, or the like). In some variants, configuration circuitry **1628** can (optionally) configure one or more of filters **1675, 1676** according to specific criteria, such as by favoring those relating to a specific pathology of interest. Alternatively or additionally, data records can be kept and distinguished that each relate to a respective user, with some users having attributes in common (e.g. age, ethnicity, genetic commonality, language, health history, or other group attribute). Such group data can be used for establishing normalcy criteria relating to a specific user/member as described herein, for example.

Operation **2288** describes causing at least the data from the occupational or leisure intercommunication to be distilled into at least one hypothesis indication (e.g. category selector **1630** indicating "respiratory," "speech," "cognitive," "memory," "vision," "infection," "intoxication" or other category **1632** having a possible relation to an apparent health status change indicated by such data). In various embodiments, the hypothesis indication(s) can include a diagnosis, prognosis, recommendation, descriptor, or other possibility of potential use to a user, diagnostician, statistician, caregiver, analyst, or the like.

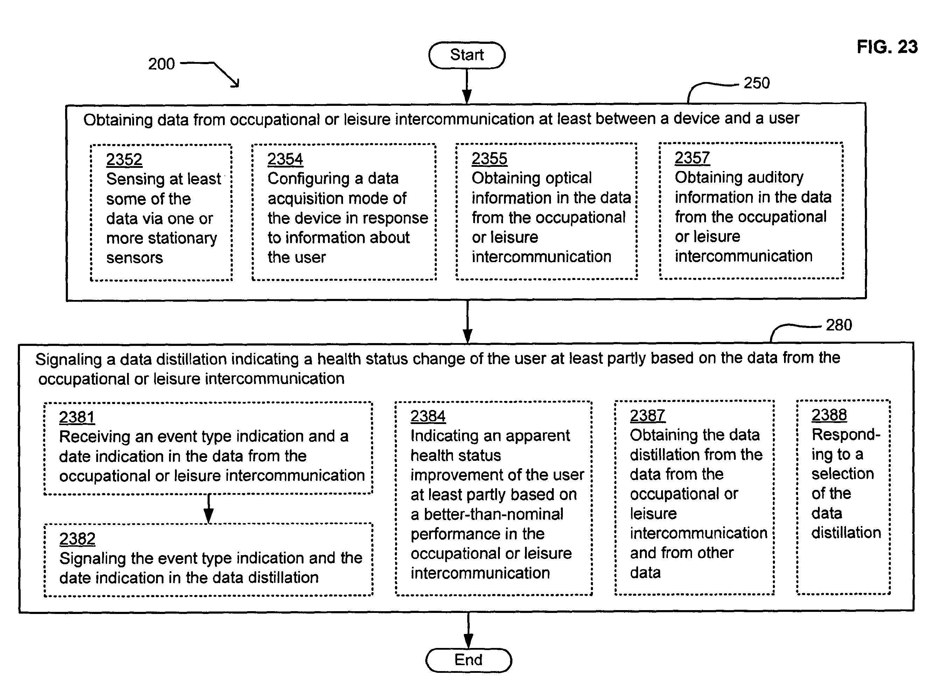

With reference now to **FIG. 23****,** there are shown several variants of the flow **200** of **FIG. 2****,** **21****,** or **22****.** Operation **250** obtaining data from occupational or leisure intercommunication at least between a device and a user may include one or more of the following operations: **2352, 2354, 2355,** or **2357.** Operation **280** signaling a data distillation indicating a health status change of the user at least partly based on the data from the occupational or leisure intercommunication may include one or more of the following operations: **2381, 2382, 2384, 2387,** or **2388.**

Operation **2352** describes sensing at least some of the data via one or more stationary sensors (e.g. sensor **113** or sensor **1712** generating data indicative of communication to or from user **130** via device **110**). This can occur, for example, in embodiments in which one or more instances of sensors **113, 1712** are stationary. Alternatively or additionally, system **1700** can be configured with a power supply **1795** operable via an electrical outlet, and optionally including one or more instances of sensors **1711, 1712, 1713,** servers **1765,** aggregators **1770,** recorders **1780,** or other elements that can incorporate motors or otherwise consume significant levels of power.

Operation **2354** describes configuring a data acquisition mode of the device in response to information about the user (e.g. category selector **1746** designating signals from microphone or other phone **1721** or the like as data **1493** for pathology-specific logic **1463** in response to a suggestion that a specified user might suffer a disorder with auditory indications). A wide variety of such disorders are well documented and generally susceptible to some degree of direct auditory detection: joint disorders, cardiological disorders, respiratory disorders, or the like. In some instances in which an anomaly is detected for which no category is apparent, aggregator **1770** can respond in a "miscellaneous capture" mode so that some or all data about the anomaly is archived for potential future analysis.

Operation **2355** describes obtaining optical information in the data from the occupational or leisure intercommunication (e.g. image analysis logic **1453** obtaining data from a camera or other sensor **113** in the user's vicinity). This can occur, for example, in embodiments in which interface **1720** and sensor **113** jointly (and each) perform operation **250** and in which one or more instances of aggregators **1770** or distillation modules **1660, 1790** perform operation **280.**

Operation **2357** describes obtaining auditory information in the data from the occupational or leisure intercommunication (e.g. auditory analysis logic **1451** obtaining data from a telephonic or other auditory signal associated with the intercommunication). The auditory signal may include speech indicating that the user is having trouble hearing, for example, or may include other sounds in the user's vicinity. In some instances, a variety of phenomena may be relevant to determinations relating to a health status change. Loud noises in a user's environment can contraindicate (or cause) an apparent health status change in some instances, for example. In some variants, sample selection logic preferentially increases an auditory or other sampling rate near anomalies, symptoms identified as significant, or other events that a diagnostician might designate as having a higher-than-nominal utility.

Operation **2381** describes receiving an event type indication and a date indication in the data from the occupational or leisure intercommunication (e.g. port **1609** receiving data including one or more dates **1621** or the like that map to one or more type indications **1622).** Such indications can include one or more instances of an interaction type indicator, a user application name, use or productivity statistics, event descriptors, or the like. Such table entry data can, in some instances, also include raw data or other matter of which some might be irrelevant, duplicative, voluminous, or otherwise unwieldy.

Operation **2382** describes signaling the event type indication and the date indication in the data distillation (e.g. filter **1608** removing some or all of the above-referenced "other" matter but passing along some or all of the dates and type indications). In some embodiments, some or all of the resulting data distillation can be presented to one or more interested parties (by display **1611** roughly in real time using flow **1200,** e.g.), with or without being captured or archived.

Operation **2384** describes indicating an apparent health status improvement of the user at least partly based on a better-than-nominal performance in the occupational or leisure intercommunication (e.g. leisure interface **111,** occupational interface **112,** or other occupational or leisure software providing data indicating a sufficiently consistent and/or dramatic improvement in a user's endurance or coordination to support a reasonable inference that the user's physical health has improved). Such an inference is unlikely to be supportable merely by showing improvement within a first few weeks of game play, however, during which time any improvements are at least as attributable to improving skills of the game. Significant improvements at a user's longstanding favorite game, however, can reasonably support an inference that a new medication or exercise regimen is working, especially if corroborated by other signs of improvement. New achievements in productivity with often-used occupational software can likewise provide significant objective evidence of a health status improvement or decline.

Operation **2387** describes obtaining the data distillation from the data from the occupational or leisure intercommunication and from other data (e.g. aggregator **1770** selectively retaining portions of data from the occupational or leisure intercommunications as well as from other interactions, as data aggregation **1773).** Alternatively or additionally, data aggregation **1773** can include (the "other") data from occupational or leisure interactions among other devices and users. In some variants, some or all of the data distillation can relate to extracting a non-probative portion **1761** from data **1763** irrespective of how much of it arises directly from the intercommunication: null event reports, "no change" or default record values, inaudible audio clips, pattern replications, solid black or white images, or the like.

Operation **2388** describes responding to a selection of the data distillation (e.g. sample selection logic **1457** activating one or more other analysis module(s) **1400** in response to one or more system selections). The user or a healthcare provider can select which modules to activate, for example, in response to which one or more portions of data **1481-1499** are selectively obtained, filtered, aggregated, analyzed, selected, evaluated, or otherwise distilled as described herein.

With reference now to **FIG. 24****,** there are shown several variants of the flow **600** of **FIG. 6****.** Operation **620** obtaining an indication of an activity status change of an application program may include one or more of the following operations: **2422, 2424, 2425,** or **2429.** Operation **690** causing a movement of data distillation code relating to physiology-indicative data in response to the indication of the activity status change of the application program may include one or more of the following operations: **2491, 2493, 2494, 2496,** or **2497.**

Operation **2422** describes receiving information about an active process of the application program (e.g. port **1861** receiving an image name, a process status, a security level, a resource identifier, an owner, a location or the like in relation to one or more processes **1845** of program **1841).** Alternatively or additionally, the information can include one or more computed estimates of quantities such as a completion time, a probability, a progress report, a resource usage status, a rate or level, an evaluation or the like relating to any thread, state information, user, task, or other aspect of program **1841.**

Operation **2424** describes receiving an output from the application program (e.g. port **1555** receiving an event code or other clinical data **1562** from program **1841).** This can occur, for example, in embodiments in which interface circuitry **1860** performs operation **620** (such as by implementing an instance of control module **1540** in interface circuitry **1860**), and in which other portions of control circuitry **1870** or common resources **1830** perform operation **690.** In some embodiments, the output can be part of what program **1841** presents at user interface **1854** or can include or otherwise reflect data in **1852** received from a patient or remote source indirectly, such as via router **1899.**

Operation **2425** describes receiving an indicator of a user of the application program (e.g. Port **1863** receiving user identification **1848,** session record **1837,** or other data indicative of one or more device users **1851** interacting with program **1841).** In some embodiments, the indicator can signal other users interacting with program **1841** or device user(s) **1851** interacting with one or more other programs so as to generate the physiology-indicative data of operation **690.**

Operation **2429** describes configuring a module with information specific to the application program (e.g. configuration circuitry **1867** modifying one or more table entries **1831** by including one or more records indicating an object name, path, current status, or other feature identifiers **1833** in association with an identifier **1832** of program **1841** or any of its components). Alternatively or additionally, in some embodiments, configuration circuitry **1867** can make or otherwise signal such configurations in response to changes in files or other records, to messages or other new data, to other signals from active programs, or to other events indicative of program activity. Many such indications are readily available from common programs, depending on the contexts in which they operate.

Operation **2491** describes loading the data distillation code relating to physiology-indicative data into a memory (e.g. memory manager **1874** copying code segment **1881** into or among one or more memory devices **1855**). This can occur, for example, in embodiments in which at least control circuitry **1870** performs operation **690,** optionally in cooperation with other portions of system **1800.** Alternatively or additionally, in an embodiment in which external system **590** of **FIG. 5** implements one or more instances of systems **1800,** network linkage **1878** can be configured to upload interaction data distillation code **517** and language data **2027** into memory **530** so that the former can be used at least in processing the latter.

Operation **2493** describes configuring a processor in response to one or more physiological expressions (e.g. task manager **1873** queuing processor **1875** to apply a filter comprising code segment **1882** associated with a segment label of "Cardio_33"). Such a physiological expression can optionally include or relate to an body part or other anatomical term, a drug or other regimen feature, a pathology, a medical phenomenon, a surgical procedure, or the like. In some embodiments, the physiological expression or logical equivalent forms a part of a name of a path, a file, a database field name, a process, a variable name, a logical expression, or the like. Alternatively or additionally, processor **1875** can (optionally) be configured by providing it with one or more code segments **1881, 1882, 1883, 1884, 1885, 1886** selected at least partly based on the physiological expression(s).

Operation **2494** describes causing the movement of the data distillation code across a network linkage (e.g. resource control circuitry **1877** requesting that code segment **1883** be transmitted via network linkage **1878**). In other instances, memory manager **1874** can be configured to cause code segment **1884** to be copied to or from one or more memory device(s) **1855** remotely via network linkage **1878** or the like. (Alternatively, of course system **1800** can be implemented entirely within one physical site.)

Operation **2496** describes selecting the data distillation code in response to an attribute of the application program (e.g. code selection logic **1879** identifying code segment **1885** with a memory address or other code-identifying information associated with program **1842**). Alternatively or additionally, some or all of the code selection information can be received from user interface **1854,** table entries **1831,** or the like. For example, an operating system may be configured to query or otherwise receive process- or self-identifying information from program **1842.**

Operation **2497** describes requesting the data distillation code relating to physiology-indicative data (e.g. request circuitry **1872** responding to physiology-indicative data in **1853** by sending request **1871** that causes code segment **1886** to arrive among a library of distillation code sources). For example, code segment **1886** can be kept in a central or regional server site until a version is needed locally, facilitating the use of up-to-date local code on demand or customized code for distilling the data in **1853.**

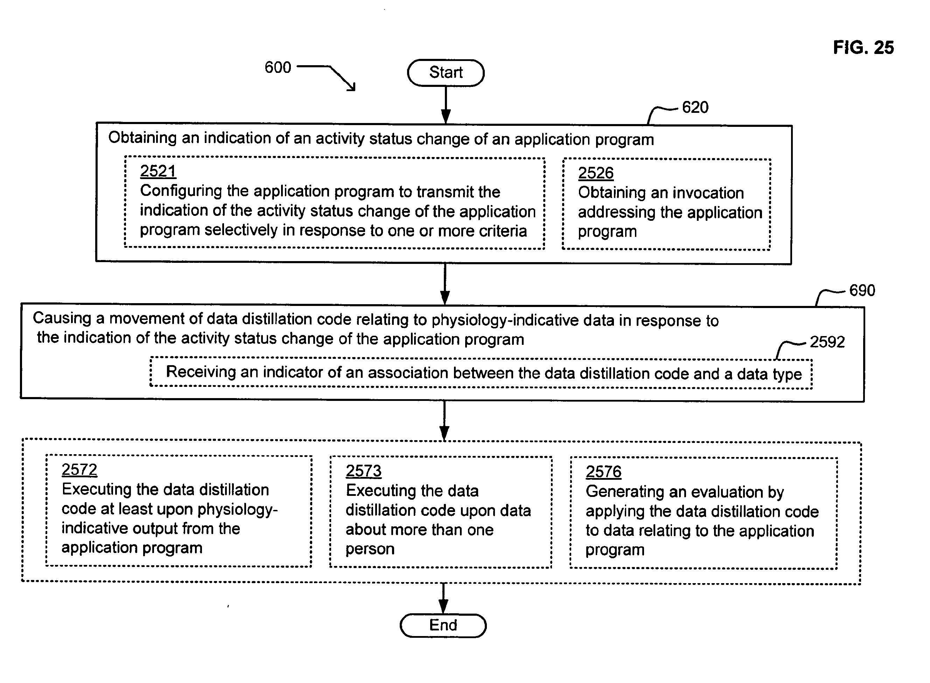

With reference now to **FIG. 25****,** there are shown several variants of the flow **600** of **FIG. 6** **or** **24****.** Operation **620** obtaining an indication of an activity status change of an application program may include one or more of the following operations: **2521** or **2526.** Operation **690** causing a movement of data distillation code relating to physiology-indicative data in response to the indication of the activity status change of the application program may include operation **2592.** Alternatively or additionally flow **600** may likewise include one or more of operations **2572, 2573,** or **2576**-each depicted after operation **690** but optionally performed concurrently with it, or in other orders.

Operation **2521** describes configuring the application program to transmit the indication of the activity status change of the application program selectively in response to one or more criteria (e.g. interface circuitry **596** configuring one or more resident programs **594** with one or more criteria **595** operable for deciding when or how to transmit an activation or deactivation signal). In some instances, for example, the one or more criteria **595** can signal a process activation, a rate of progress, an "activate" command, or the like, for example, or a core initialization that includes loading at least part of program **1841.** This can occur, for example, in embodiments in which external system **590** performs at least operation **620.**

Operation **2526** describes obtaining an invocation addressing the application program (e.g. sensor **1897** receiving an invocation addressing one or more of programs **1841, 1842, 1843).** This can occur, for example, in embodiments in which one or more common resources **1830** perform operation **620.** In some implementations, such a trigger signal or other invocation can itself indicate a nominal, likely or recent activity status change of the program(s) to which it is addressed.

Operation **2592** describes receiving an indicator of an association between the data distillation code and a data type receiving an indicator of an association between the data distillation code and a data type (e.g. at least some association circuitry **1300** receiving an update or other logic indicating one or more instances of image analysis linkage(s) **1313** linking "jpg," "mpeg," or other image-containing data type(s) 1333 with a corresponding reference to data distillation code such as access information **1353).** Alternatively or additionally, the association can contain literal implementations of some or all function(s) **1343** appropriate for the associated data type(s) **1333.** In some variants, one or more other linkages can be received from association circuitry **1300,** for example, by control circuitry **1870** or the like in performing operation **690.** In various embodiments, such linkages can include one or more instances of auditory analysis linkage(s) **1311,** cardiological analysis linkage(s) **1312,** kinesthetic analysis linkage(s) **1315,** interval detection linkage(s) **1319,** or the like.

Operation **2572** describes executing the data distillation code at least upon physiology-indicative output from the application program (e.g. processor **511** applying optical data distillation code **513** to sample image output from a webcam program, a digital photograph upload utility, an e-mail application, or the like). In some embodiments, user stimuli data **2022** may contain optical images, for example, to show what a user found disturbing or to identify blind portions of a user's field of view (the left side or center, e.g.). User action data **2023** may likewise contain diagnostically relevant optical images, for example, to suggest what a user was doing shortly before suffering a seizure or going into shock. Such images may likewise comprise cognitive indications **2026,** for example, showing that a user showed a pattern of drowsiness or agitation. In such cases, the images may likewise contain or accompany timestamps, device identifiers, regimens, or similar incidental data **2028** to describe circumstances under which pathological indications or other physiology-indicative output was captured. Other kinds of program output data **2038** can include data arising from observing patients, data from auser's parent or other caregiver, surreptitious data, or the like as exemplified herein.

Operation **2573** describes executing the data distillation code upon data about more than one person (e.g. processor **511** executing auditory data distillation code **515** upon data from several parties, for example, to establish a normal range of coughing frequencies for a demographic group). Such norms can be useful for initial screening for a wide variety of pathologies. For example, such norms may form a basis for associating a specific user with pathological data **2024** (e.g. detailed data about asthma, bronchitis, and allergic response detection criteria downloaded in response to a preliminary indication of the user's abnormally frequent coughing). Alternatively or additionally, a workforce or other technology-literate community can, in some embodiments, be screened to identify one or more members likely to be suitable for a vaccine or other experimental, preventive or corrective treatment.

Operation **2576** describes generating an evaluation by applying the data distillation code to data relating to the application program (e.g. processor **511** or other circuitry generating one or more data distillations **529** that include a ranking, a level indication, a category, or the like). In some embodiments, the evaluation can be generated using one or more instances of neurological analysis logic **1461,** cardiological analysis logic **1462,** pathology-specific logic **1463,** anomaly detection logic **1464,** or any of the other items described herein in relation to data distillation. Alternatively or additionally, the evaluation can be partly or wholly based on one or more instances of configuration data **2021,** user stimuli data **2022,** user action data **2023,** pathological data **2024,** language data **2027,** incidental data **2028,** biometric data **2029** or the like.

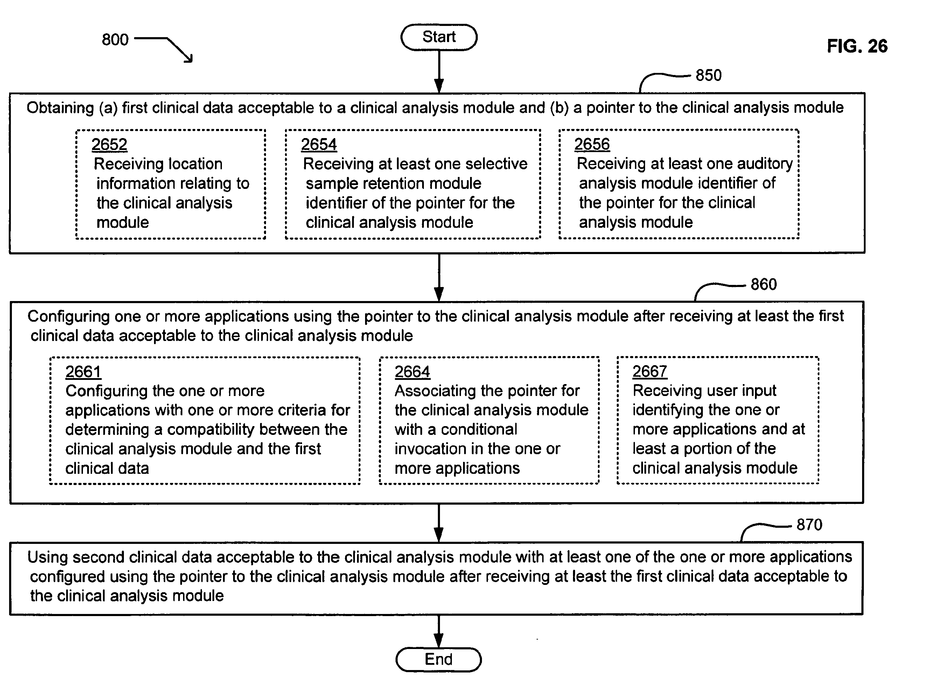

With reference now to **FIG. 26****,** there are shown several variants of the flow **800** of **FIG. 8****.** Operation 850 obtaining (a) first clinical data acceptable to a clinical analysis module and (b) a pointer to the clinical analysis module may include one or more of the following operations: **2652, 2654,** or **2656.** Operation **860**-configuring one or more applications using the pointer to the clinical analysis module after receiving at least the first clinical data acceptable to the clinical analysis module-may include one or more of the following operations: **2661, 2664,** or **2667.** Many variants of operation 870 using second clinical data acceptable to the clinical analysis module with at least one of the one or more applications configured using the pointer to the clinical analysis module after receiving at least the first clinical data acceptable to the clinical analysis module are also provided herein: in which the data is used by a distillation module as described herein in variants of flow **200,** for example, or in which performance-indicative data is used according to variants of flow **1000.**

Operation **2652** describes receiving location information relating to the clinical analysis module (e.g. port **1553** receiving a path, pointer, or other location information **1541** relating to image analysis logic **1453**). This can occur, for example, in embodiments in which interface module **1550** performs operation **850.** Alternatively or additionally, location information **1541** can include references to auditory analysis logic **1451,** performance analysis logic **1456,** or the like. In some embodiments, the location information can be received locally (e.g. user interface **1551** receiving the information from a local operator).

Operation **2654** describes receiving at least one selective sample retention module identifier of the pointer for the clinical analysis module (e.g. port **1554** receiving a logical address or other location information relating to sample selection logic **1457).** In some embodiments, such selection logic can comprise selective sample retention logic, indexing or ranking logic, a data compressor or other filter, or other selective mechanism as described with reference to other figures herein.

Operation **2656** describes receiving at least one auditory analysis module identifier of the pointer for the clinical analysis module (e.g. port **1556** receiving a data object name, module name, or other reference data **1557** relating specifically to auditory analysis logic **1451**). In some embodiments, one or more ports can likewise receive information sufficient to identify one or more other clinical analysis module(s) **720** or analysis module(s) **1400.** Alternatively or additionally, the "first" clinical data can be received from sensor(s) **725** or **1548.**

Operation **2661** describes configuring the one or more applications with one or more criteria for determining a compatibility between the clinical analysis module and the first clinical data (e.g. resource manager **1574** providing "B" type app(s) **1592** with file types, protocol version numbers, or the other filter parameters **1595** for generating an indication of whether performance analysis logic **1456** can apparently process "Type 2" portion **1567** at least partly in response to descriptive data **1565**). This can occur, for example, in embodiments in which configuration circuitry **1570** performs operation **860** (alone or jointly with interface module **1550,** e.g.). The descriptive data relating to clinical data **1563** can, for example, include an owner, an input device identifier, a sample or summary of clinical data **1563,** or the like. A relation between such descriptive information and clinical data **1563** can be obtained, for example, by including the information within the data, by a categorization or known arrangement of the data, by applying data format evaluation or other criteria, or the like. In some embodiments, a preliminary indication of compatibility can be inferred in response to an error message, a type list, an allowable range of values, or the like. Alternatively or additionally, such criteria can be derived by trying to process the data portions with "A" type app(s) **1591,** and by indicating an apparent compatibility in response to a suitable interval without an error report or the like, or to some other indication of successful processing.

Operation **2664** describes associating the pointer for the clinical analysis module with a conditional invocation in the one or more applications (e.g. association logic **1575** associating pointer **1542** with a jump operation that is only performed if a variable is TRUE, in the one or more "A" type apps **1591).** In some embodiments, of course, the invocation can reside in a non-branching code block that is only performed if a specific condition exists (e.g. within a local program branch of "A" type apps **1591,** performed if a computed result is equal to zero). Alternatively or additionally, the invocation can request processing in a remote task or instance of analysis modules **1400,** for example, or queue or otherwise trigger some instance of such a module.

Operation **2667** describes receiving user input identifying the one or more applications and at least a portion of the clinical analysis module (e.g. input device **1573** receiving typed data, menu selections, voice data, or the like specifying an app type or other descriptors, a pathological or other physiological term, a data type, a task attribute, or other information distinguishing the selected tasks, applications or code functionality from others). Those skilled in the art will recognize a variety of ways of including or excluding "A" type app(s) **1591,** for example, from the set to be configured. The user input can be received from user interface **715** via network interface **730,** in some embodiments, such as those in which primary system **700** implements control module **1540** as described above. Alternatively or additionally, the user input can include pointer **1543** or other indications of any clinical analysis module(s) to be enabled.

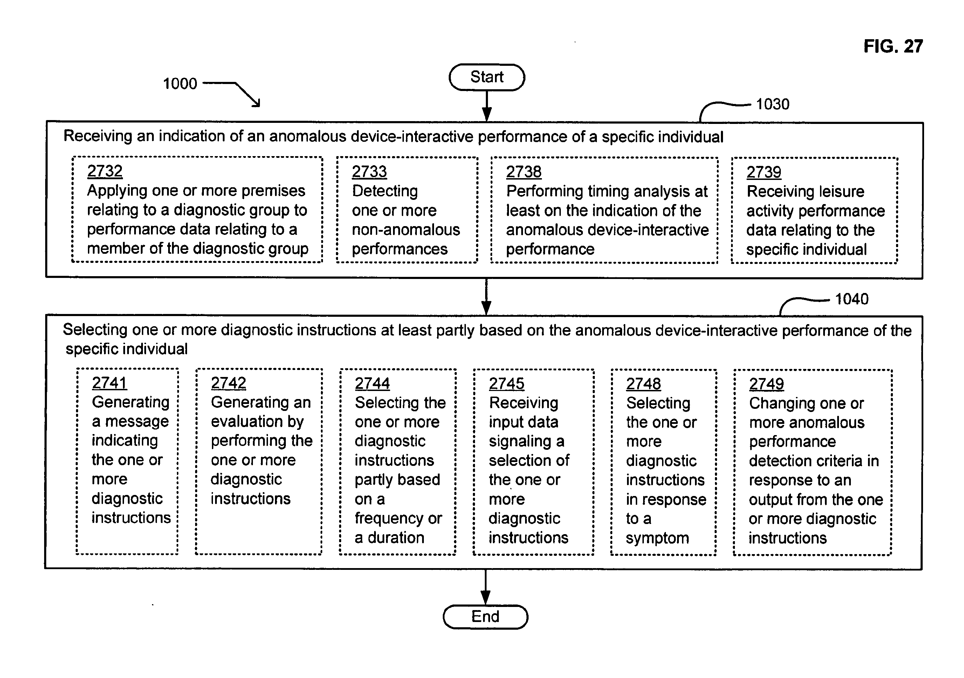

With reference now to **FIG. 27****,** there are shown several variants of the flow **1000** of **FIG. 10****.** Operation **1030** receiving an indication of an anomalous device-interactive performance of a specific individual may include one or more of the following operations: **2732, 2733, 2738** or **2739.** Operation **1040** selecting one or more diagnostic instructions at least partly based on the anomalous device-interactive performance of the specific individual may include one or more of the following operations: **2741, 2742, 2744, 2745, 2748,** or **2749.**

Operation **2732** describes applying one or more premises relating to a diagnostic group to performance data relating to a member of the diagnostic group (e.g. normative logic **1958** applying a pulse rate range for 42-year-old women to evaluate whether a specific 42-year-old woman has a normal pulse rate for her age). The diagnostic group can be bounded by age range, gender or other genetic attribute, symptom or other medical status, activity type, affiliation, interface type, linguistic or cultural norms, geography, or the like.

Operation **2733** describes detecting one or more non-anomalous performances (e.g. anomaly detection logic **1464** or the like indicating data **1484** as "normal" or otherwise non-anomalous). In various embodiments, such logic can implement one or more of analysis module(s) **1400** of **FIG. 14****,** for example. In some embodiments, a portion of the non-anomalous data can be retained by sample selection logic **1457,** for example, as samples or other indications representative of a normal condition of the individual(s). Alternatively or additionally, sample selection logic **1457** can preferentially select anomaly-indicative samples in various ways in light of teachings herein. In this way, for example, normal fluctuations can be objectively and economically distinguished from apparent transitional events or trends (suddenly or gradually increasing an addictive behavior by 50% or more, for example, such as smoking or online game play).

Operation **2738** describes performing timing analysis at least on the indication of the anomalous device-interactive performance (e.g. interval detection logic **1469** or the like determining whether a reaction time or the like was within a normal range). This can occur, for example, in embodiments in which device **900** or intake module **1940** includes one or more instances of analysis module(s) **1400,** in which intake module **1940** performs operation **1030,** and in which analysis module **1960** or other portions of system **1900** perform other operations of flow **1000.** Determining that a timing aspect of the device-interactive performance was abnormal can, in some embodiments, trigger an inference that the performance was anomalous. In some variants, a priori knowledge of the specific individual (e.g. age, past performance, or the like) can enhance the accuracy of such normalcy thresholds, as described herein.

Operation **2739** describes receiving leisure activity performance data relating to the specific individual (e.g. port **941** receiving data relating to individual **930** or some other individual performing some device-interactive leisure activity). The leisure activity can include a conversation via a telephonic device or keyboard, playing an instrument or computer game, going for a drive, or some other leisure activity performed by interacting with a system like that of device **900.** Such data can be useful in many ways as described herein, such as by a processor-based system that can record it or otherwise detect it in some fashion.

Operation **2741** describes generating a message indicating the one or more diagnostic instructions (e.g. message generator **1964** signaling a clinician to administer a patient questionnaire, a blood test, or some other diagnostic instrument that depends at least partly on anomaly indications **956**). Alternatively or additionally, the message can direct one or more analysis modules to be performed upon performance data **1952** or the like to facilitate a diagnosis, in light of teachings herein.

Operation **2742** describes generating an evaluation by performing the one or more diagnostic instructions (e.g. evaluation module **1969** generating a computed variance, a sample of data selected as representative, a phrase, a percentile, or the like to describe qualitative or quantitative attributes of the anomaly or other aspect of the performance). In some embodiments, for example, the evaluation can include a text value of "+," an error message, "irregular," a form paragraph, or the like.

Operation **2744** describes selecting the one or more diagnostic instructions partly based on a frequency or a duration (e.g. branch logic **967** selecting instructions **964** in lieu of instructions **965** because a computed result indicating that a computed duration was negative). This can occur, for example, when the negative result indicates that one event happened before another (e.g. a deadline expiring before the specific individual responds). A kind of positive result can likewise occur, such as when the individual succeeds with an unusually high frequency. A diagnostician can fairly infer a cognitive or sensory improvement from a suddenly higher frequency of success, for example, which can be helpful for evaluating whether a new regimen was helpful. In some embodiments, of course, branch logic **967** can likewise be configured to decide among access object(s) **968** or instructions **964** based on durations, frequencies, or other time-related computations. Those skilled in the art will recognize many such embodiments in light of teachings herein.

Operation **2745** describes receiving input data signaling a selection of the one or more diagnostic instructions (e.g. one or more instances of interfaces **940** detecting a user action indicating a selection validation indicator or selection indicating one or more analysis module(s) **1400).** In some embodiments, for example, such a selection can designate a menu option indicating an instruction series. Alternatively or additionally, the selection can be performed before operation **1030** is complete and so that the selection controls how later-received anomaly indications will be treated.

Operation **2748** describes selecting the one or more diagnostic instructions in response to a symptom (e.g. pathology-specific logic **1463** selecting kinesthetic analysis logic **1465** or the like in response to images and auditory data indicating that a patient may be suffering from increasing symptoms of a joint disorder). The symptoms may include a a popping sound or facial or verbal expression of pain in conjunction with a joint motion, in some embodiments. Others of analysis module(s) **1400** may likewise be selected in appropriate circumstances, such as by selecting cardiological analysis logic **1462** in response to an indication of potential heart trouble. Alternatively or additionally, a user interface as described herein can show a physician a list of available analysis modules relating to a symptom of pain evident to the physician in a video clip of a patient.

Operation **2749** describes changing one or more anomalous performance detection criteria in response to an output from the one or more diagnostic instructions (e.g. some instance of anomaly detection logic **1464** incrementally expanding a normal range defined in data **2036** in response to an indication of too many false positives in anomaly indication(s) **956**). Conversely a set of normal values defined in data **2036** can be incrementally reduced in response to an indication of too many false negatives, in some embodiments, so that configuration circuitry as described herein can cause anomaly detection logic **1464** to draw closer to an optimal sensitivity adaptively.

With reference now to **FIG. 28****,** there are shown several variants of the flow **1000** of **FIG. 10** or **27****.** Operation **1030** receiving an indication of an anomalous device-interactive performance of a specific individual may include one or more of the following operations: **2831, 2833, 2834, 2836** or **2838.** Alternatively or additionally flow **1000** may likewise include one or more of operations **2861, 2864, 2866, 2868,** or **2869** each depicted after operation **1040** but optionally performed concurrently with it, or in other orders.

Operation **2831** describes detecting a performance anomaly at least by comparing a scalar performance indicator with a threshold (e.g. comparator **1951** indicating whether performance data **1952** indicates an apparent anomaly in response to measuring a recent success rate of 60% in comparison to a historical success range of 72% to 84%). Likewise a performance anomaly can be recognized in response to an average power level increasing significantly (by at least about a decibel in some contexts, e.g.). A change of these types can optionally be detected by a applying a minimum or maximum threshold to a scalar performance indicator such as a ratio, an absolute percentage change, a computed variance, a number of metric units, or the like. Those skilled in the art will recognize a wide variety of scalar thresholds, and groups thereof, suitable for distinguishing anomalies from insignificant fluctuation in light of these teachings.

Operation **2833** describes detecting an anomaly after the anomalous device-interactive performance of the specific individual ends (e.g. scanning logic **1956** indicating which members of a population of test subjects exhibit a recognizable performance anomaly). The anomaly may be initially recognized as matching one or more performance pattern(s) **1957** essentially in the same manner that human beings can be observed to recognize any of a variety of patterns, often unconsciously. Fatigue-indicative patterns, for example, can include yawning, sluggishness, slow reactions, diminished functional performance, or the like. Many such patterns can readily be recognized readily available image recognition techniques and applied in light of teachings herein.

In some embodiments, scanning logic **1956** can recognize such patterns in data, such as by applying image analysis logic **1453** to data **2034** from an earlier device-interactive performance of the specific individual(s). In some embodiments, at least some raw data from many individuals is archived for later analysis by subsequent versions of one or more instances of analysis modules **1960.**

Operation **2834** describes detecting an anomaly during the anomalous device-interactive performance of the specific individual (e.g. monitoring logic **1953** detecting a sign of apparently severe distress in a user's interaction with or through a handheld device during the interaction or perhaps at least within a diagnostically appropriate period of the interaction). This can occur, for example, in embodiments in which intake module **1940** performs operation **1030.** Depending on one or more pathologies of the individual, such periods may comprise a few minutes, a few hours, a few days, a few months, or even longer. In some embodiments herein, such interactions can include a conversation, a transaction or session, or the like. Such signs (sobbing, screaming, fleeing, or the like) can be recognized from audio, image, or motion data in the device in some embodiments described herein, or from other sensors nearby.

Operation **2836** describes applying at least auditory processing of the indication of the anomalous device-interactive performance of the specific individual (e.g. detection logic **1954** including auditory analysis logic **1451** or the like configured for distilling at least some category or other indication of a telephonic conversation or other device-interactive performance as described herein). In some embodiments, the auditory processing can include a filter that preferentially retains vocal-range frequency signals, for example. Alternatively or additionally, a filter can be used for removing time segments of audio data with no recognizable sounds. Those skilled in the art will recognize in light of these teachings that such techniques tend to enhance a performance of the retained data (such as can be indicated by a signal-to-noise ratio, as a speech density in words or recognizable inflections per unit of storage or display, or the like).

Operation **2838** describes configuring a device according to an attribute of the specific individual (e.g. storage manager **1942** loading media **1944** with one or more analysis module(s) **1400** selected for use for the specific individual, or one or more instances of values **1945** therefrom). In some embodiments, the device configuration can be customized according to the individual's gender, age, symptom(s), or other medical history. Such analysis module(s) **1400** can be selected programmatically by a pathology or the like, for example, or according to authorizations, default values, or other information such as may be provided by physician or other authorized caregiver.

Operation **2861** describes obtaining information confirming an identity of the specific individual after the anomalous device-interactive performance of the specific individual (e.g. subject identification logic **1468** recording photographic or other biometric data, requesting a password, or otherwise authenticating an identity of one or more of users **130, 335,** party **1102,** or specific individual **930**). Such authentications or the like can occur, for example, in any of several variants of flows **200, 400, 1000, 1200** described herein as an appropriate response to receiving data or taking other actions described herein, generally to affirm or otherwise assure the appropriateness of responsive actions.

Operation **2864** describes performing a transmission of or a reception of an instruction sequence including at least the one or more diagnostic instructions (e.g. update module **1962** transmitting or receiving code that includes a sequence of diagnostic instructions). This can implement a local or remote update of analysis module(s) **1400** as described herein, or of applications containing analysis module(s) **1400,** in response to the anomalous device-interactive performance(s) of the specific individual.

Operation **2866** describes executing the one or more diagnostic instructions (e.g. processor **1927** executing code selected at least partly based on the device-interactive performance). In some embodiments, the type or timing of detected anomalies can at least partly control when or how the diagnostic instruction(s) are executed. In some embodiments, for example, an anomaly of interest primarily for research purposes can trigger a lower priority execution of the pertinent code than that of a crisis-indicative anomaly.

Operation **2868** describes causing the one or more diagnostic instructions to generate a diagnosis (e.g. processor **1928** activating one or more analysis modules **1400** resulting in at least one diagnosis of one or more attributes of the specific individual). In an embodiment in which one or more analysis modules **1400** includes an ability to recognize the indication of the anomalous performance as a false positive, for example, the diagnosis be null, "normal" or the like. This can occur, for example, in embodiments in which the one or more analysis modules **1400** can infer from a fuller analysis that the false positive apparently resulted from a data glitch or other cause unrelated to the specific individual.

Operation **2869** describes requesting the one or more diagnostic instructions (e.g. request logic **1967** requesting one or more instructions selected in operation **1040** from a remote device, not shown). Alternatively or additionally, a library or other collection that includes such instruction(s) can be requested before or during operation **1040,** and the outcome of the selection can be used for deciding which such instruction(s) to execute. In some embodiments, the instruction(s) selected can be obtained and then implemented in applications as described herein.

With reference now to **FIG. 29****,** there are shown several variants of the flow **1200** of **FIG. 12****.** Operation **1250** signaling a decision whether to notify a first party at least by applying a first screen to one or more health-status-indicative updates relating to a second party may include one or more of the following operations: **2951, 2953, 2957,** or **2958.** Operation **1280** signaling a decision whether to notify a third party at least by applying a second screen to the one or more health-status-indicative updates relating to the second party may include one or more of the following operations: **2982, 2983, 2986,** or **2988.**

Operation **2951** describes determining whether the one or more health-status-indicative updates indicate a new symptom (e.g. sensor **1410** detecting that a symptom-indicative parameter has changed by a magnitude large enough to support an inference that the "second" party has a new symptom). This can occur, for example, in embodiments in which one or more instances of sensors **1410, 1421** or other logic **1451-1469** can perform operation **1250** and in which one or more instances of distribution circuitry **1440** or transmitters **1430** can perform operation **1280.**

Those skilled in the art will recognize that in some contexts any patient status change will constitute a "new symptom" inference: yes/no determinations of whether the second party/user has a pulse, can respond, or the like, for example. In some embodiments, sensor **1410** can implement one or more criteria **1414** for making such determinations: by computing a difference between or otherwise comparing a past-symptom or range vector **1415** with a recent-status vector **1416,** for example. If the "first" party's notification profile includes a reference to a just-manifested symptom (or to all new symptoms), distribution circuitry **1440** can implement such profiles in one or more distribution modes **1444.**

Operation **2953** describes determining whether the one or more health-status-indicative updates indicate a crisis (e.g. sensor **1421** applying one or more physician-or other expert-defined thresholds or other crisis-indicative criteria **1422** to determine whether or which parties **1101-1104** should be notified). The "first" party may be a physician or ambulance service, for example, for an audible or other significant indication that a heart patient or other at-risk "second" party may be suffering a heart attack, a seizure, a condition causing tremors or other observable symptoms, an auto wreck, complications from a surgery, or the like. In some distribution modes **1441-1449** of "third" parties, the inclusion or responses of one or more "first" parties may contraindicate the decision whether to notify a "third" party.

Operation **2957** describes associating the first screen with a distribution list including at least the first party (e.g. one or more instances of linkages **1311-1319** associating one or more data type(s) **1331-1339** with corresponding access information **1351-1359** authorizing notification to or other access by the "first" party). This can occur, for example, in embodiments in which the first party includes one or more of parties **1101, 1102;** in which invocation module **1130** has access to or otherwise implements one or more linkages **1311-1319** of association circuitry **1300** or other association logic **1576,** in which at least some decision circuitry **1141, 1142** performs operation **1250,** and in which at least some other decision circuitry **1142, 1143** or external circuitry **1190** performs operation **1280.**

Operation **2958** describes determining whether the one or more health-status-indicative updates indicate a health deterioration (e.g. sensor **1427** applying one or more first-party-defined thresholds or other criteria **1428** to some or all of data **1491-1499** to determine whether a potential health deterioration is apparent therefrom). Sensor **1427** can be configured, for example, by party **1105** defining screen A **1151** so as to be notified whenever data **1493** indicates a body temperature (of party **1101,** e.g.) above 39° Celsius or a pulse rate of 140 BPM, or further increases therefrom. Substantially all such notices can, for some pathologies, signal a health status deterioration. Many types of health status deteriorations are detectable in embodiments herein, such as can manifest in a pathology-indicative quantity or the like becoming abnormal or more abnormal. In some variants, for example, an apparent health status decline of one or more users **130, 335** is detected at least partly based on one or more worsening or other worse-than-nominal performances in a surreptitiously observed or occupational or leisure interaction as described herein. Those skilled in the art can recognize such quantities as they reflect deteriorations in the "second" party or user in relation to his/her normal range or that of one or more others. In some cases a hypothesis relating to the deterioration (e.g. drunkenness, exhaustion, overdose, injury, illness, or the like) can be obtained from context information (conversation, e.g.) or directly (from a caregiver who identifies a pathology, e.g.).

Operation **2982** describes notifying the first party and the third party of a result of the second screen (e.g. distribution circuitry **1440** applying mode **1449** to notify two or more caregivers or the like). This can be desirable, for example, in contexts in which the second screen detects an unusually significant event: a stroke, arrhythmia, an auto accident, a physical attack or the like apparent bearing upon a health status of at least the "second" party.

Operation **2983** describes transmitting to the third party the one or more health-status-indicative updates including at least a selected sample of raw data (e.g. invoker **1432** causing party **1103** to receive remotely archived or other user-entered data, video or audio segments, or other such raw data **2039** significantly indicative of a health status evaluation of party **1102** relying on the raw data). This can occur, for example, in embodiments in which some or all analysis module(s) **1400** or data-containing media **2030** available to primary system **1130** are distributed across more than one site. Such raw data can ordinarily be of immediate interest to the "third" party, for example, to corroborate or perhaps at least elaborate upon an evaluation or other "second" screen triggering the notification decision.

Operation **2986** describes notifying the second party and the third party of a result of the second screen (e.g. distribution circuitry **1440** applying mode **1447** ). A distribution list, party inclusion or selection criteria, or like can be used as mode **1447** by which distribution circuitry **1440** notifies at least the "second" and "third" parties. In some embodiments a patient (as the "second" party, e.g.) wishes to notify a third party automatically in response to a debilitating event (as the "second" screen, e.g.) so that the patient's chosen third party will receive prompt notice. Operation **2986** can cause such a patient to receive similar notice (if conscious), serving as a roughly contemporaneous indication that the chosen third party is being notified.

Operation **2988** describes archiving the decision whether to notify the third party (e.g. aggregator **1770** implementing filter **1771** by recording at least a partial list of who was or should be notified) . This can occur, for example, in embodiments in which configuration module **1408** provides or adapts filter **1771** according to selections received from one or more users (as the "second" party) described herein. The archiving can optionally occur before or in some other temporal relation to such notification, in some embodiments. Alternatively or additionally, some variants of operation **2988** can be performed in relation to a "first" party (or some other user) and combined with one or more other user-related operations described herein.

With reference now to **FIG. 30****,** there are shown several variants of the flow **400** of **FIG. 4****.** Operation **410** receiving health-status-indicative data surreptitiously captured from an interaction between a device and a user may include one or more of the following operations: **3012, 3015,** or **3019.** Operation **440** applying one or more data extraction criteria to the health-status-indicative data surreptitiously captured from the interaction between the device and the user may include one or more of the following operations: **3043, 3044, 3046, 3048,** or **3049.**

Operation **3012** describes receiving leisure activity performance data of the health-status-indicative data (e.g. port **1754** receiving at least user game moves, scores, screen captures, social interactions, browsing selections or the like of which some might reasonably be expected to indicate any substantial emotional or personality shifts, or similar health status data). This can occur, for example, in embodiments in which one or more instances of input modules **371** or interfaces **1610, 1720** perform operation **410,** in which one or more instances of distillation modules **1660, 1790** or extraction modules **333** perform operation **440,** or in which system **1600** is implemented as a portable device or other stand-alone system, or within an embodiment of system **1700.**

Operation **3015** describes receiving the health-status-indicative data substantially contemporaneously with the interaction (e.g. recorder **1780** surreptitiously capturing image or audio data from an interaction between an infant and a toy). In some variants, recorder can include filter **1781** operable for pausing the recorder or otherwise excluding or marking less-desirable data: data that is not surreptitious or is unrelated to the interaction between the device and the user. Alternatively or additionally, recorder can include filter **1782** operable for excluding or identifying (at least some) data that is not indicative of health status, such as by activating the recording only in response to an expression of a symptom.

Operation **3019** describes configuring at least the device to capture the health-status-indicative data surreptitiously (e.g. configuration logic **1718** adapting one or more instances of interfaces **1610, 1720** of devices **330** so that the user will not be conscious of being monitored). In some variants in which user **335** consents to being monitored, for example, configuration logic **1718** causes at most a subtle reminder (or no reminder) to be presented to the user real time, for most or all of the capture period(s).

Operation **3043** describes selecting extraction logic in response to input from an interface of the device (e.g. selection logic **1691** selecting one or more extraction modes **1695-1697** or the like in response to input **1612** from interface **1610**). Extraction mode **1695** can include a clip selection mode or the like, for example, in response to a performance anomaly or like indication that some video or audio data of input **1612** may be more likely to be relevant than a remainder of the video or audio data. Alternatively or additionally, in a wireless implementation or other context in which a distribution bottleneck can occur, control logic can instead implement mode **1696,** by which data types and times are provided at least initially in lieu of video or audio footage.

Operation **3044** describes applying one or more extraction modes selected in response to data-type-indicative information (e.g. extraction logic **1778** applying mode **1697** selected at least partly in response to "JPG," "language," "MP3" or other data type or format category **1632** from category selector **1630**). Such modes can optionally be selected in response to one or more predetermined attributes of the user such as at association with some data **2031-2039** obtained earlier as described herein (optionally via data processor **1650** or the like).

Operation **3046** describes selecting the one or more data extraction criteria at least partly based on information relating to the user (e.g. selection logic **1692** selecting translator **1671** or the like for use in or with filter **1674** or other extraction logic, in response to a priori or detected language data **2037** indicating that the user speaks or writes in Lithuanian). This can sometimes matter in a context in which such criteria can facilitate further analysis as described herein (which may not otherwise function in Lithuanian, for example). Other such helpful user-related information can include one or more instances of health status (age, gender, genetic background, medical history, current medications, or the like), location, software or device usage habits, sleep habits, or the like.

Operation **3048** describes selecting the one or more data extraction criteria at least partly based on a menu selection (e.g. selection logic **1694** selecting at least criterion **1698** according to an option selection received as input **1612**). This can occur, for example, in embodiments in which system **1600** includes one or more instances of interfaces **1720** or recorders **1780,** in which at least interface **1720** performs operation **410,** and in which one or more instances of control logic **1690** or distillation modules **1660** perform operation **440.**

Operation **3049** describes deciding whether to signal a party partly based on the health-status-indicative data surreptitiously captured from the interaction between the device and the user (e.g. selection logic **1693** selecting the "first" party of **FIG. 12** in response to an indication that this party should be notified of surreptitiously captured data). In the even that even surreptitiously captured data objectively indicates that the user is becoming more manic, for example, providing this indication directly to the user's doctor (by e-mail or automatic voice message, e.g.) may help the doctor be more prompt in adjusting the user's dosage (of lithium or the like, e.g.). The decision whether to signal the party can also depend on one or more of a time of day, the party's location or other status information, the user's location or other status information, medical or other historical information, other data relating to the interaction or to the device, or the like.

The foregoing detailed description has set forth various embodiments of the devices and/or processes via the use of block diagrams, flowcharts, and/or examples. Insofar as such block diagrams, flowcharts, and/or examples contain one or more functions and/or operations, it will be understood by those within the art that each function and/or operation within such block diagrams, flowcharts, or examples can be implemented, individually and/or collectively, by a wide range of hardware, software, firmware, or virtually any combination thereof. In one embodiment, several portions of the subject matter described herein may be implemented via Application Specific Integrated Circuits (ASICs), Field Programmable Gate Arrays (FPGAs), digital signal processors (DSPs), or other integrated formats. However, those skilled in the art will recognize that some aspects of the embodiments disclosed herein, in whole or in part, can be equivalently implemented in integrated circuits, as one or more computer programs running on one or more computers (e.g., as one or more programs running on one or more computer systems), as one or more programs running on one or more processors (e.g., as one or more programs running on one or more microprocessors), as firmware, or as virtually any combination thereof, and that designing the circuitry and/or writing the code for the software and or firmware would be well within the skill of one of skill in the art in light of this disclosure. In addition, those skilled in the art will appreciate that the mechanisms of the subject matter described herein are capable of being distributed as a program product in a variety of forms, and that an illustrative embodiment of the subject matter described herein applies regardless of the particular type of signal bearing medium used to actually carry out the distribution. Examples of a signal bearing medium include, but are not limited to, the following: a recordable type medium such as a floppy disk, a hard disk drive, a Compact Disc (CD), a Digital Video Disk (DVD), a digital tape, a computer memory, etc.; and a transmission type medium such as a digital and/or an analog communication medium (e.g., a fiber optic cable, a waveguide, a wired communications link, a wireless communication link, etc.).

In a general sense, those skilled in the art will recognize that the various embodiments described herein can be implemented, individually and/or collectively, by various types of electro-mechanical systems having a wide range of electrical components such as hardware, software, firmware, or virtually any combination thereof; and a wide range of components that may impart mechanical force or motion such as rigid bodies, spring or torsional bodies, hydraulics, and electro-magnetically actuated devices, or virtually any combination thereof. Consequently, as used herein "electro-mechanical system" includes, but is not limited to, electrical circuitry operably coupled with a transducer (e.g., an actuator, a motor, a piezoelectric crystal, etc.), electrical circuitry having at least one discrete electrical circuit, electrical circuitry having at least one integrated circuit, electrical circuitry having at least one application specific integrated circuit, electrical circuitry forming a general purpose computing device configured by a computer program (e.g., a general purpose computer configured by a computer program which at least partially carries out processes and/or devices described herein, or a microprocessor configured by a computer program which at least partially carries out processes and/or devices described herein), electrical circuitry forming a memory device (e.g., forms of random access memory), electrical circuitry forming a communications device (e.g., a modem, communications switch, or optical-electrical equipment), and any non-electrical analog thereto, such as optical or other analogs. Those skilled in the art will also appreciate that examples of electro-mechanical systems include but are not limited to a variety of consumer electronics systems, as well as other systems such as motorized transport systems, factory automation systems, security systems, and communication/computing systems. Those skilled in the art will recognize that electro-mechanical as used herein is not necessarily limited to a system that has both electrical and mechanical actuation except as context may dictate otherwise.

In a general sense, those skilled in the art will recognize that the various aspects described herein which can be implemented, individually and/or collectively, by a wide range of hardware, software, firmware, or any combination thereof can be viewed as being composed of various types of "electrical circuitry." Consequently, as used herein "electrical circuitry" includes, but is not limited to, electrical circuitry having at least one discrete electrical circuit, electrical circuitry having at least one integrated circuit, electrical circuitry having at least one application specific integrated circuit, electrical circuitry forming a general purpose computing device configured by a computer program (e.g., a general purpose computer configured by a computer program which at least partially carries out processes and/or devices described herein, or a microprocessor configured by a computer program which at least partially carries out processes and/or devices described herein), electrical circuitry forming a memory device (e.g., forms of random access memory), and/or electrical circuitry forming a communications device (e.g., a modem, communications switch, or optical-electrical equipment). Those having skill in the art will recognize that the subject matter described herein may be implemented in an analog or digital fashion or some combination thereof.

Those skilled in the art will recognize that it is common within the art to describe devices and/or processes in the fashion set forth herein, and thereafter use engineering practices to integrate such described devices and/or processes into image processing systems. That is, at least a portion of the devices and/or processes described herein can be integrated into an image processing system via a reasonable amount of experimentation. Those having skill in the art will recognize that a typical image processing system generally includes one or more of a system unit housing, a video display device, a memory such as volatile and non-volatile memory, processors such as microprocessors and digital signal processors, computational entities such as operating systems, drivers, and applications programs, one or more interaction devices, such as a touch pad or screen, control systems including feedback loops and control motors (e.g., feedback for sensing lens position and/or velocity; control motors for moving/distorting lenses to give desired focuses. A typical image processing system may be implemented utilizing any suitable commercially available components, such as those typically found in digital still systems and/or digital motion systems.

Those skilled in the art will recognize that it is common within the art to describe devices and/or processes in the fashion set forth herein, and thereafter use engineering practices to integrate such described devices and/or processes into data processing systems. That is, at least a portion of the devices and/or processes described herein can be integrated into a data processing system via a reasonable amount of experimentation. Those having skill in the art will recognize that a typical data processing system generally includes one or more of a system unit housing, a video display device, a memory such as volatile and non-volatile memory, processors such as microprocessors and digital signal processors, computational entities such as operating systems, drivers, graphical user interfaces, and applications programs, one or more interaction devices, such as a touch pad or screen, and/or control systems including feedback loops and control motors (e.g., feedback for sensing position and/or velocity; control motors for moving and/or adjusting components and/or quantities). A typical data processing system may be implemented utilizing any suitable commercially available components, such as those typically found in data computing/communication and/or network computing/communication systems.

Those skilled in the art will recognize that it is common within the art to implement devices and/or processes and/or systems in the fashion(s) set forth herein, and thereafter use engineering and/or business practices to integrate such implemented devices and/or processes and/or systems into more comprehensive devices and/or processes and/or systems. That is, at least a portion of the devices and/or processes and/or systems described herein can be integrated into other devices and/or processes and/or systems via a reasonable amount of experimentation. Those having skill in the art will recognize that examples of such other devices and/or processes and/or systems might include - as appropriate to context and applicationall or part of devices and/or processes and/or systems of (a) an air conveyance (e.g., an airplane, rocket, hovercraft, helicopter, etc.), (b) a ground conveyance (e.g., a car, truck, locomotive, tank, armored personnel carrier, etc.), (c) a building (e.g., a home, warehouse, office, etc.), (d) an appliance (e.g., a refrigerator, a washing machine, a dryer, etc.), (e) a communications system (e.g., a networked system, a telephone system, a Voice over IP system, etc.), (f) a business entity (e.g., an Internet Service Provider (ISP) entity such as Comcast Cable, Quest, Southwestern Bell, etc), or (g) a wired/wireless services entity such as Sprint, Cingular, Nextel, etc.), etc.

All of the above-mentioned U.S. patents, U.S. patent application publications, U.S. patent applications, foreign patents, foreign patent applications and non-patent publications referred to in this specification and/or listed in any Application Data Sheet, are incorporated herein by reference, to the extent not inconsistent herewith.

One skilled in the art will recognize that the herein described components (e.g., steps), devices, and objects and the discussion accompanying them are used as examples for the sake of conceptual clarity and that various configuration modifications are within the skill of those in the art. Consequently, as used herein, the specific exemplars set forth and the accompanying discussion are intended to be representative of their more general classes. In general, use of any specific exemplar herein is also intended to be representative of its class, and the non-inclusion of such specific components (e.g., steps), devices, and objects herein should not be taken as indicating that limitation is desired.

Although users **130, 335, 1851** are typically shown and described herein each as a single illustrated figure, those skilled in the art will appreciate that such users may be representative of a human user, a robotic user (e.g., computational entity), and/or substantially any combination thereof (e.g., a user may be assisted by one or more robotic agents). In addition, each such user, as set forth herein, although shown as a single entity may in fact be composed of two or more entities. Those skilled in the art will appreciate that, in general, the same may be said of "sender" and/or other entity-oriented terms as such terms are used herein.

An aspect described herein provides a method comprising: obtaining an indication of an activity status change of an application program; and causing a movement of data distillation code relating to physiology-indicative data in response to the indication of the activity status change of the application program.

An aspect described herein includes a system comprising: means for obtaining an indication of an activity status change of an application program; and means for causing a movement of data distillation code relating to physiology-indicative data in response to the indication of the activity status change of the application program.

An aspect described herein includes a system comprising: circuitry for obtaining an indication of an activity status change of an application program; and circuitry for causing a movement of data distillation code relating to physiology-indicative data in response to the indication of the activity status change of the application program.

An aspect described herein include a method comprising: obtaining (a) first clinical data acceptable to a clinical analysis module and (b) a pointer to the clinical analysis module;configuring one or more applications using the pointer to the clinical analysis module after receiving at least the first clinical data acceptable to the clinical analysis module; and using second clinical data acceptable to the clinical analysis module with at least one of the one or more applications configured using the pointer to the clinical analysis module after receiving at least the first clinical data acceptable to the clinical analysis module.

An aspect described herein includes a system comprising: means forobtaining (a) first clinical data acceptable to a clinical analysis module and (b) a pointer to the clinical analysis module; means for configuring one or more applications using the pointer to the clinical analysis module after receiving at least the first clinical data acceptable to the clinical analysis module; and means for using second clinical data acceptable to the clinical analysis module with at least one of the one or more applications configured using the pointer to the clinical analysis module after receiving at least the first clinical data acceptable to the clinical analysis module.

An aspect described herein includes a system comprising: circuitry forobtaining (a) first clinical data acceptable to a clinical analysis module and (b) a pointer to the clinical analysis module; circuitry for configuring one or more applications using the pointer to the clinical analysis module after receiving at least the first clinical data acceptable to the clinical analysis module; and circuitry for using second clinical data acceptable to the clinical analysis module with at least one of the one or more applications configured using the pointer to the clinical analysis module after receiving at least the first clinical data acceptable to the clinical analysis module.

A system, method, computer program product, and carrier are described for obtaining an indication of an activity status change of an application program and causing a movement of data distillation code relating to physiology-indicative data in response to the indication of the activity status change of the application program; or for obtaining (a) first clinical data acceptable to a clinical analysis module and (b) a pointer to the clinical analysis module, configuring one or more applications using the pointer to the clinical analysis module after receiving at least the first clinical data acceptable to the clinical analysis module, and using second clinical data acceptable to the clinical analysis module with at least one of the one or more applications configured using the pointer to the clinical analysis module after receiving at least the first clinical data acceptable to the clinical analysis module.

An aspect described herein includes a method comprising:receiving an indication of an anomalous device-interactive performance of a specific individual; and selecting one or more diagnostic instructions at least partly based on the anomalous device-interactive performance of the specific individual.

An aspect described herein includes a system comprising: means for receiving an indication of an anomalous device-interactive performance of a specific individual; and means for selecting one or more diagnostic instructions at least partly based on the anomalous device-interactive performance of the specific individual.

An aspect described herein includes a system comprising: circuitry for receiving an indication of an anomalous device-interactive performance of a specific individual; and circuitry for selecting one or more diagnostic instructions at least partly based on the anomalous device-interactive performance of the specific individual.

An aspect described herein includes a method comprising: signaling a decision whether to notify a first party at least by applying a first screen to one or more health-status-indicative updates relating to a second party; and signaling a decision whether to notify a third party at least by applying a second screen to the one or more health-status-indicative updates relating to the second party.

An aspect described herein includes a system comprising: means for signaling a decision whether to notify a first party at least by applying a first screen to one or more health-status-indicative updates relating to a second party; and means forsignaling a decision whether to notify a third party at least by applying a second screen to the one or more health-status-indicative updates relating to the second party.

An aspect described herein includes a system comprising: circuitry forsignaling a decision whether to notify a first party at least by applying a first screen to one or more health-status-indicative updates relating to a second party; and circuitry forsignaling a decision whether to notify a third party at least by applying a second screen to the one or more health-status-indicative updates relating to the second party.

An aspect described herein includes a method comprising:receiving health-status-indicative data surreptitiously captured from an interaction between a device and a user; and applying one or more data extraction criteria to the health-status-indicative data surreptitiously captured from the interaction between the device and the user.

An aspect described herein includes a system comprising: means for receiving health-status-indicative data surreptitiously captured from an interaction between a device and a user; and means for applying one or more data extraction criteria to the health-status-indicative data surreptitiously captured from the interaction between the device and the user.

An aspect described herein includes a system comprising: circuitry for receiving health-status-indicative data surreptitiously captured from an interaction between a device and a user; and circuitry for applying one or more data extraction criteria to the health-status-indicative data surreptitiously captured from the interaction between the device and the user.

An aspect described herein includes a method comprising:obtaining data from occupational or leisure intercommunication at least between a device and a user; and signaling a data distillation indicating a health status change of the user at least partly based on the data from the occupational or leisure intercommunication.

An aspect described herein includes a system comprising: means forobtaining data from occupational or leisure intercommunication at least between a device and a user; and means forsignaling a data distillation indicating a health status change of the user at least partly based on the data from the occupational or leisure intercommunication.

An aspect described herein includes a system comprising: circuitry forobtaining data from occupational or leisure intercommunication at least between a device and a user; and circuitry forsignaling a data distillation indicating a health status change of the user at least partly based on the data from the occupational or leisure intercommunication.

With respect to the use of substantially any plural and/or singular terms herein, those having skill in the art can translate from the plural to the singular and/or from the singular to the plural as is appropriate to the context and/or application. The various singular/plural permutations are not expressly set forth herein for sake of clarity.

The herein described subject matter sometimes illustrates different components contained within, or connected with, different other components. It is to be understood that such depicted architectures are merely example, and that in fact many other architectures can be implemented which achieve the same functionality. In a conceptual sense, any arrangement of components to achieve the same functionality is effectively "associated" such that the desired functionality is achieved. Hence, any two components herein combined to achieve a particular functionality can be seen as "associated with" each other such that the desired functionality is achieved, irrespective of architectures or intermedial components. Likewise, any two components so associated can also be viewed as being "operably connected", or "operably coupled", to each other to achieve the desired functionality, and any two components capable of being so associated can also be viewed as being "operably couplable", to each other to achieve the desired functionality. Specific examples of operably couplable include but are not limited to physically mateable and/or physically interacting components and/or wirelessly interactable and/or wirelessly interacting components and/or logically interacting and/or logically interactable components.

While particular aspects of the present subject matter described herein have been shown and described, it will be apparent to those skilled in the art that, based upon the teachings herein, changes and modifications may be made without departing from the subject matter described herein and its broader aspects and, therefore, the appended claims are to encompass within their scope all such changes and modifications as are within the true spirit and scope of the subject matter described herein. Furthermore, it is to be understood that the invention is defined by the appended claims. It will be understood by those within the art that, in general, terms used herein, and especially in the appended claims (e.g., bodies of the appended claims) are generally intended as "open" terms (e.g., the term "including" should be interpreted as "including but not limited to," the term "having" should be interpreted as "having at least," the term "includes" should be interpreted as "includes but is not limited to," etc.). It will be further understood by those within the art that if a specific number of an introduced claim recitation is intended, such an intent will be explicitly recited in the claim, and in the absence of such recitation no such intent is present. For example, as an aid to understanding, the following appended claims may contain usage of the introductory phrases "at least one" and "one or more" to introduce claim recitations. However, the use of such phrases should not be construed to imply that the introduction of a claim recitation by the indefinite articles "a" or "an" limits any particular claim containing such introduced claim recitation to inventions containing only one such recitation, even when the same claim includes the introductory phrases "one or more" or "at least one" and indefinite articles such as "a" or "an" (e.g., "a" and/or "an" should typically be interpreted to mean "at least one" or "one or more"); the same holds true for the use of definite articles used to introduce claim recitations. In addition, even if a specific number of an introduced claim recitation is explicitly recited, those skilled in the art will recognize that such recitation should typically be interpreted to mean at least the recited number (e.g., the bare recitation of "two recitations," without other modifiers, typically means at least two recitations, or two or more recitations). Furthermore, in those instances where a convention analogous to "at least one of A, B, and C, etc." is used, in general such a construction is intended in the sense one having skill in the art would understand the convention (e.g., " a system having at least one of A, B, and C" would include but not be limited to systems that have A alone, B alone, C alone, A and B together, A and C together, B and C together, and/or A, B, and C together, etc.). In those instances where a convention analogous to "at least one of A, B, or C, etc." is used, in general such a construction is intended in the sense one having skill in the art would understand the convention (e.g., " a system having at least one of A, B, or C" would include but not be limited to systems that have A alone, B alone, C alone, A and B together, A and C together, B and C together, and/or A, B, and C together, etc.). It will be further understood by those within the art that virtually any disjunctive word and/or phrase presenting two or more alternative terms, whether in the description, claims, or drawings, should be understood to contemplate the possibilities of including one of the terms, either of the terms, or both terms. For example, the phrase "A or B" will be understood to include the possibilities of "A" or "B" or "A and B."

With respect to the appended claims, those skilled in the art will appreciate that recited operations therein may generally be performed in any order. Examples of such alternate orderings may include overlapping, interleaved, interrupted, reordered, incremental, preparatory, supplemental, simultaneous, reverse, or other variant orderings, unless context dictates otherwise. With respect to context, even terms like "responsive to," "related to," or other past-tense adjectives are generally not intended to exclude such variants, unless context dictates otherwise.

While various aspects and embodiments have been disclosed herein, other aspects and embodiments will be apparent to those skilled in the art. The various aspects and embodiments disclosed herein are for purposes of illustration and are not intended to be limiting, with the true scope and spirit being indicated by the following claims.

## Claims

1. A method comprising:
obtaining an indication of an activity status change of an application program; and
causing a movement of data distillation code relating to physiology-indicative data in response to the indication of the activity status change of the application program.

2. The method of claim 1 in which said obtaining comprises at least one of:
receiving information about an active process of the application program; receiving an output from the application program;
receiving an indicator of a user of the application program;
configuring a module with information specific to the application program;
configuring the application program to transmit the indication of the activity status change of the application program selectively in response to one or more criteria; or
obtaining an invocation addressing the application program..

3. The method of claim 1 or claim 2 in which said causing a movement comprises at least one of:
loading the data distillation code relating to physiology-indicative data into a memory;
configuring a processor in response to one or more physiological expressions;causing the movement of the data distillation code across a network linkage;selecting the data distillation code in response to an attribute of the application program;requesting the data distillation code relating to physiology-indicative data; orreceiving an indicator of an association between the data distillation code and a data type.

4. The method of any preceding claim further comprising at least one of:
executing the data distillation code at least upon physiology-indicative output from the application program;executing the data distillation code upon data about more than one person;generating an evaluation by applying the data distillation code to data relating to the application program.

5. A method comprising:
obtaining (a) first clinical data acceptable to a clinical analysis module and (b) a pointer to the clinical analysis module;
configuring one or more applications using the pointer to the clinical analysis module after receiving at least the first clinical data acceptable to the clinical analysis module; and
using second clinical data acceptable to the clinical analysis module with at least one of the one or more applications configured using the pointer to the clinical analysis module after receiving at least the first clinical data acceptable to the clinical analysis module.

6. The method of claim 5 in which said obtaining comprises at least one of:
receiving location information relating to the clinical analysis module;
receiving at least one selective sample retention module identifier of the pointer for the clinical analysis module; or
receiving at least one auditory analysis module identifier of the pointer for the clinical analysis module.

7. The method of claim 5 or claim 6 in which said configuring comprises:
configuring the one or more applications with one or more criteria for determining a compatibility between the clinical analysis module and the first clinical data;
associating the pointer for the clinical analysis module with a conditional invocation in the one or more applications;
receiving user input identifying the one or more applications and at least a portion of the clinical analysis module.

8. A system comprising means for performing the method of any preceding claim.

9. The system of claim 8, wherein said means comprise:
means for obtaining an indication of an activity status change of an application program; and
means for causing a movement of data distillation code relating to physiology-indicative data in response to the indication of the activity status change of the application program.

10. The system of claim 8, wherein said means comprise:
means for obtaining (a) first clinical data acceptable to a clinical analysis module and (b) a pointer to the clinical analysis module;
means for configuring one or more applications using the pointer to the clinical analysis module after receiving at least the first clinical data acceptable to the clinical analysis module; and
means for using second clinical data acceptable to the clinical analysis module with at least one of the one or more applications configured using the pointer to the clinical analysis module after receiving at least the first clinical data acceptable to the clinical analysis module.

11. The system of any of claims 8 to 10, wherein said means comprise circuitry.
